# FASCICULE DE BREVET EUROPEEN

(11) **EP 0 542 609 B1**
(45) Date de publication et mention de la délivrance du brevet: **07.09.1994**
(21) Numéro de dépôt: 92403014.1
(22) Date de dépôt: 09.11.1992
(51) Int. Cl.: C07D 215/38, C07D 409/12, C07D 491/04, A61K 31/47, C07D 471/04, C07D 498/04

(54) **3-Sulfonylamino-2-(1H)-quinoléinone et des dérivés 7-aza comme angatonistes d'acides aminés excitateurs**
3-Sulfonylamino-2-(1H)-Chinolinone und deren 7-Azaderivate als anregende Aminosäuren Antagonisten
3-sulfonylamino-2-(1H)-quinolinones and 7-aza derivatives as excitatory amino acids antagonists

(30) Priorité: 14.11.1991 FR 9113977
(43) Date de publication de la demande: 19.05.1993
(73) Titulaire: ADIR ET COMPAGNIE, 92415 Courbevoie Cédex (FR)
(72) Inventeur: Cordi, Alex, F-92150 Suresnes (FR); Desos, Patrice, F-92400 Courbevoie (FR); Lepagnol, Jean, F-28210 Chaudon (FR); Randle, John, F-92100 Boulogne Billancourt (FR)

(56) Documents cités:
- EP-A- 0 386 839
- US-A- 3 066 145

## Description

La présente invention concerne de nouveaux dérivés de 3-sulfonylamino-2-(1H)-quinoléinone, leur procédé de préparation et les compositions pharmaceutiques qui les contiennent.

Quelques dérivés de 2-(1H)-quinoléinone ont été décrits dans la littérature. C'est le cas, par exemple, des composés décrits par C. ALABASTER et coll. (J. Med. Chem., 31, 2048-2056, 1988) qui sont des stimulateurs cardiaques, de ceux décrits par F. BAHR et coll. (Pharmazie, 36, H.10, 1981), ou des composés décrits dans le brevet US 3,066,145 qui possèdent des propriétés anticonvulsives et tranquillisantes.

Les composés décrits dans la présente invention, outre le fait qu'ils soient nouveaux, présentent des propriétés pharmacologiques particulièrement intéressantes : ce sont de puissants inhibiteurs des phénomènes liés à l'hyperactivation des aminoacides excitateurs.

L'acide L-glutamique et l'acide L-aspartique ont la capacité d'activer les neurones du système nerveux central et de nombreux travaux ont démontré que ces aminoacides excitateurs (AAE) répondent aux critères définissant un neurotransmetteur. C'est pourquoi la modulation des événements neuronaux liés à ces AAE apparait être une cible intéressante pour le traitement des maladies neurologiques.

En effet, il a été prouvé que la libération exagérée des AAE et l'hyperstimulation de leurs récepteurs serait une des causes de la dégénérescence neuronale que l'on observe dans l'épilepsie, la démence sénile ou les accidents vasculaires cérébraux. Mais actuellement, le nombre de maladies neurodégénératives dans lesquelles les AAE sont étroitement impliqués ne cesse de grandir (chorée de Huntington, schizophrénie, sclérose amyotrophique latérale) (Mc GEER E.G. et al., Nature, 263, 517-519, 1976 ; SIMON R. et al., Science, 226, 850-852, 1984).

De plus, s'il est certain que l'hyperactivation de la neurotransmission aux AAE exerce des effets neurotoxiques, l'activation normale de celle-ci facilite les performances mnésiques et cognitives (LYNCH G. & BAUDRY M., Science, 224, 1057-1063, 1984 ; ROTHMAN S.M. & OLNEY J.W., Trends in Neuro Sci., 10, 299-302, 1987). Du point de vue pharmacologique et thérapeutique, il convient donc de ne s'opposer qu'aux stimulations pathologiques tout en respectant le niveau d'activation physiologique.

Les récepteurs aux AAE à localisation post- et présynaptique ont été classés en 4 groupes en fonction de l'affinité et des effets électrophysiologiques et/ou neurochimiques de ligands spécifiques :
. récepteur NMDA (N-méthyl-D-aspartate) associé à un canal ionique perméable aux cations mono- et divalents dont le calcium mais qui est bloqué par le magnésium. L'accumulation du calcium dans la cellule serait une des causes de la mort neuronale. L'ouverture du canal NMDA est régulée par plusieurs sites associés au récepteur et en particulier est favorisée par la glycine dont l'effet est strychnine-insensible. Ce site glycine constitue l'une des cibles importantes pour moduler l'activation du récepteur NMDA.
. récepteur AMPA (α-amino-3-hydroxy-5-méthyl-4-isoxazole) propionique acide associé à un canal ionique perméable aux cations monovalents dont le sodium. L'activation de ce canal entrainerait une prédépolarisation membranaire.
. récepteur kainate dont les caractéristiques ioniques sont proches du récepteur AMPA mais qui en diffère par les niveaux de conductance et de désensibilisation. Toutefois, de nombreuses études tendent à prouver que le récepteur AMPA et le récepteur kainate ont d'étroites analogies structurales et fonctionnelles et constituent une même famille réceptorielle (KEINANEN K. et al., Science, 249, 556-560, 1990).
. récepteur ACPD (amino-cyclopentane-dicarboxylique acide) appelé récepteur métabotropique car non couplé à un canal ionique.

L'activation des récepteurs ionotropiques par les AAE ouvre les canaux ioniques et notamment permet l'entrée de sodium qui dépolarise la cellule. Cette première phase qui implique le récepteur AMPA, conduit alors à l'hyperactivation du récepteur NMDA et à l'accumulation massive de calcium (BLAKE J.F. et al., Neurosci. Letters₇ 89, 182-186, 1988 ; BASHIR Z.I. et al., Nature, 349, 156-158, 1991).

Les composés de la présente invention visent donc de manière originale à s'opposer aux effets excitateurs et toxiques des AAE par un double mécanisme :
- en bloquant l'activation initiale du récepteur à l'AMPA/kainate,
- en modulant l'hyperactivation du récepteur NMDA par blocage du site glycine.

Ce double mécanisme s'avère potentiellement différent de ceux jusqu'alors décrits pour les agents s'opposant aux effets des AAE puisque ces agents n'ont qu'un seul mécanisme d'action, spécifique pour le récepteur AMPA ou pour le récepteur NMDA ou pour le site glycine.

Les composés de la présente invention sont donc utiles en tant qu'inhibiteurs des phénomènes pathologiques notamment neurotoxiques liés à l'hyperactivation des voies de neurotransmission aux aminoacides excitateurs.

Ils sont donc des agents thérapeutiques potentiels pour le traitement des maladies neurologiques et psychiques impliquant ces aminoacides : maladies dégénératives aigües ou chroniques, telles que l'accident vasculaire cérébral, le traumatisme spinal, la sclérose amotrophique latérale, la maladie d'Alzheimer ou la schizophrénie.

Plus spécifiquement, la présente invention concerne les composés de formule (I) : 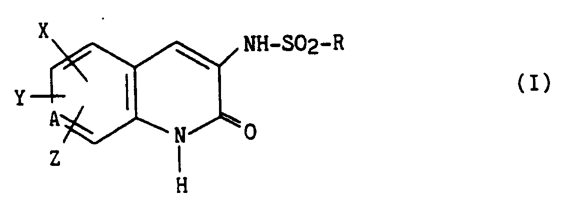
dans laquelle :
- X, Y, Z :: - identiques ou différents, représentent un atome d'hydrogène, un atome d'halogène, un groupement nitro, cyano, azido, trihalogénométhyle, alkyle (C₁-C₆) linéaire ou ramifié, alkoxy (C₁-C₆) linéaire ou ramifié, amino (substitué ou non par un groupement acyl (C₁-C₆) linéaire ou ramifié),
- ou bien, lorsqu'ils sont situés sur deux carbones adjacents, forment avec les atomes de carbone auxquels ils sont attachés, un cycle phényle ou un hétérocycle à 5 ou 6 chaînons comportant de 1 à 3 hétéroatomes, préférentiellement un cycle 1,2,5-oxadiazole,
- R: représente un groupement alkyle (C₁-C₆) linéaire ou ramifié, trihalogénométhyle, phényle (substitué ou non par un ou plusieurs atomes d'halogène ou groupements alkyle (C₁-C₆) linéaire ou ramifié, trihalogénométhyle, alkoxy (C₁-C₆) linéaire ou ramifié), 2-thiényle (substitué ou non par un atome d'halogène), naphtyle ou styryle,
- A: représente un radical CH ou un atome d'azote,
leurs énantiomères, diastéréoisomères et épimères ainsi que leurs sels d'addition à une base pharmaceutiquement acceptable.

L'invention s'étend également au procédé de préparation des composés de formule (I) caractérisé en ce que l'on utilise comme produit de départ un composé de formule (II) : 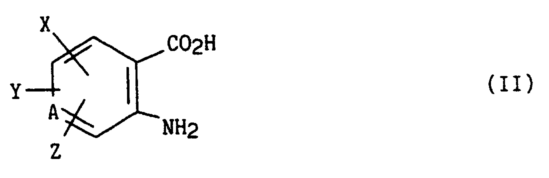
dans laquelle X, Y, Z et A ont la même signification que dans la formule (I),
que l'on réduit selon la nature des substituants X, Y et Z ,
soit en milieu aprotique en présence d'hydrure de lithium aluminium, d'hydrure d'aluminium, de diborane ou des complexes de borane,
soit en milieu protique acide lorsque l'on utilise le cyanoborohydrure de sodium,
pour conduire à un alcool de formule (III) : 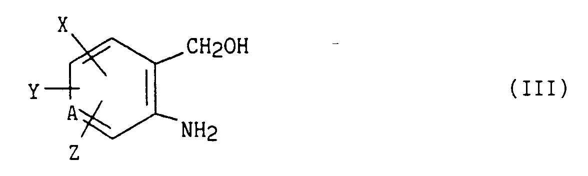
dans laquelle X, Y, Z et A ont la même signification que dans la formule (I),
que l'on oxyde à l'aide d'un oxyde métallique dans un solvant inerte, d'un halogénate de métal alcalin dans un solvant protique ou d'un chlorure d'acide dans le diméthylsulfoxyde,
pour conduire à l'adéhyde de formule (IV) : 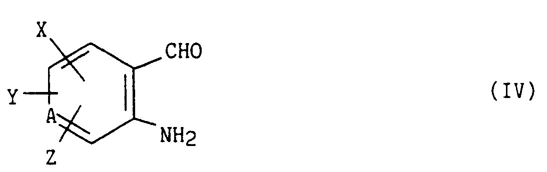
dans laquelle X, Y, Z et A ont la même signification que dans la formule (I),
qui est alors condensé :
1°/ 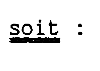 avec un malonate d'alkyle de formule (V), en milieu protique, en présence d'un alcoolate de métal alcalin, d'une amine tertiaire ou d'un hydroxyde de métal alcalin : 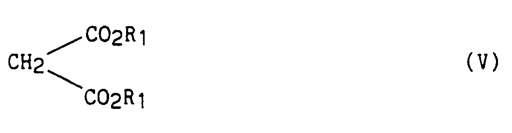 dans laquelle R₁ représente un groupement alkyle (C₁-C₆) linéaire ou ramifié, substitué ou non par un ou plusieurs groupements phényle,
   pour conduire à une 2-(1H)-quinoléinone de formule (VI) : 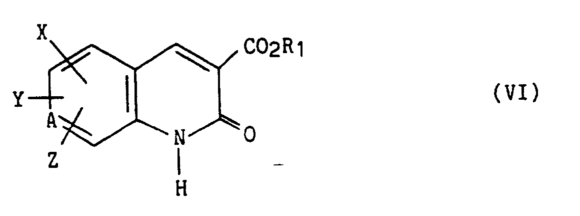 dans laquelle X, Y, Z, A et R₁ ont la même signification que précédemment, qui est protégée :
   - par réaction avec un tétrafluoroborate de trialkyloxonium dans de l'éther ou le dichlorométhane,
   - ou par réaction, dans un solvant inerte ou sans, d'un oxyhalogénure de phosphore, d'un pentahalogénure de phosphore, d'un halogénure de thionyle, de phosgène, de thiophosgène, de phosgène imminium ou de trimère de phosgène, suivie d'une réaction de la 2-halogénoquinoléine ainsi formée avec un alkoxyde de métal alcalin de formule R'₁OM (dans laquelle R'₁ a la même signification que R₁ et M représente un métal alcalin), en milieu alcoolique,
   - ou par réaction avec l'hydrure de sodium, le butyllithium, un alcoolate de métal alcalin en présence d'un agent d'alkylation de formule R'₁V (dans laquelle R'₁ a la même signification que précédemment et V est un groupe partant),
   pour conduire à la 2-alkoxyquinoléine de formule (VII) : 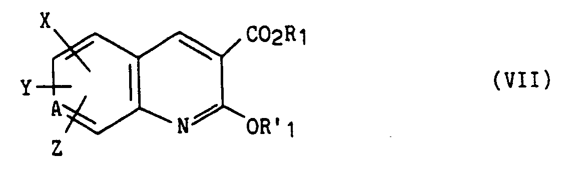 dans laquelle X, Y, Z, A, R₁ et R'₁ ont la même signification que précédemment,
   qui est hydrolysée en présence d'un hydroxyde de métal alcalin en milieu aqueux ou hydroalcoolique,
   pour conduire à l'acide de formule (VIII) : 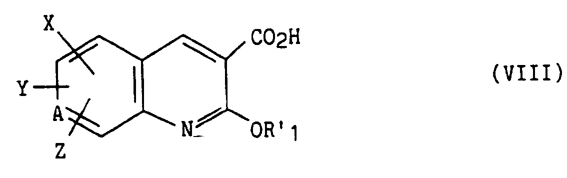 dans laquelle X, Y, Z, A et R'₁ ont la même signification que précédemment,
   qui est :
   a transformé en azoture d'acide correspondant :
      - par activation de la fonction acide sous forme d'anhydride ou d'halogènure d'acide puis réaction avec de l'azoture de sodium dans un solvant inerte,
      - ou par réaction, dans un solvant inerte, avec de l'azoture de diphénoxyphosphoryl en présence d'une amine tertiaire,
   b réarrangé, par chauffage, dans un solvant inerte, en milieu anhydre en additionnant à la fin du dégagement gazeux un excès d'un alcool de formule R''₁OH (dans laquelle R''₁ a la même signification que R₁) ou par chauffage direct dans l'alcool R''₁OH,
   pour conduire au carbamate correspondant de formule (IX) : 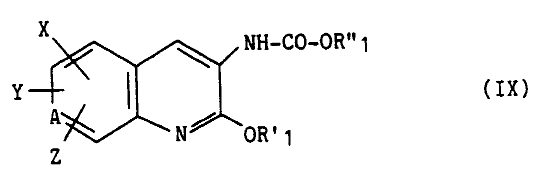 dans laquelle X, Y, Z, A, R'₁, R''₁ ont la même signification que précédemment,
   qui est soit hydrolysé en milieu acide anhydre, soit hydrogéné en milieu anhydre par de l'hydrogène en présence d'un catalyseur,
   pour conduire à l'amine de formule (X) : 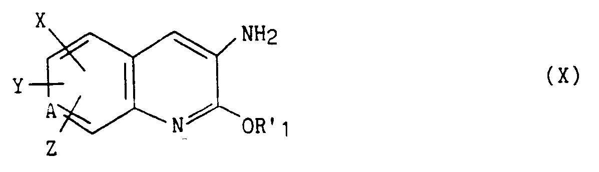 dans laquelle X, Y, Z, A et R'₁ ont la même signification que précédemment,
   qui subit une sulfonylation par réaction avec un halogénure ou un anhydride de sulfonyle de formules respectives RSO₂W ou (RSO₂)₂O (dans lesquelles R a la même signification que dans la formule (I) et W représente un atome d'halogène), en présence d'une amine tertiaire, en milieu aprotique,
   pour conduire à la sulfonamide de formule (XI) : 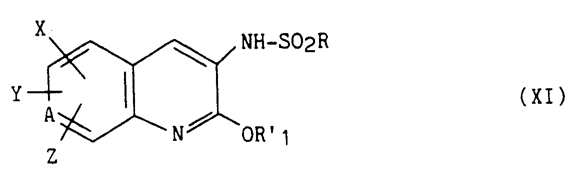 dans laquelle x, Y, Z, A, R et R'₁ ont la même signification que précédemment,
   qui est déprotégée, selon la nature du groupement R'₁, par chauffage en milieu aqueux acide ou par hydrogénation catalytique,
   pour conduire au composé de formule (I),
2°/ 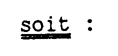 avec un nitroacétate d'alkyle en milieu protique, en présence d'un alcoolate de métal alcalin, d'une amine tertiaire ou d'un hydroxyde de métal alcalin, ou en milieu inerte à haut point d'ébullition en présence d'une amine tertiaire ou secondaire au reflux du solvant, pour conduire à la 3-nitro-2-(1H)-quinoléinone de formule (XII) : 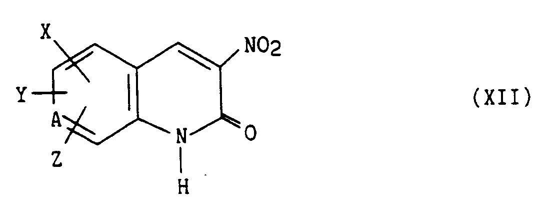 dans laquelle X, Y, Z et A ont la même signification que dans la formule (I),
   qui est protégée, selon les mêmes techniques que celles décrites pour la protection de la quinoléinone de formule (VI) en 2-alkoxyquinoléine de formule (VII),
   pour conduire à la 2-alkoxyquinoléine de formule (XIII) : 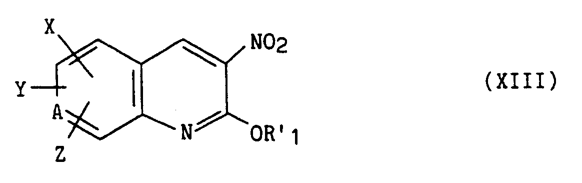 dans laquelle X, Y, Z, A et R'₁ ont la même signification que dans la formule (I),
   dont le groupe nitro est réduit en présence de chlorure d'étain dans un alcool, d'hydrosulfite de sodium dans l'eau ou par hydrogénation catalytique,
   pour conduire à l'amine de formule (X) définie précédemment,
   qui subit la sulfonylation puis la déprotection décrites plus haut permettant d'obtenir un composé de formule (I),
3°/ 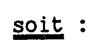 avec un dérivé de la glycine protégée de formule (XIV), activé sous forme d'anhydride, d'azoture, de cyanure ou d'halogénure correspondant, en milieu inerte, en présence de triéthylamine : 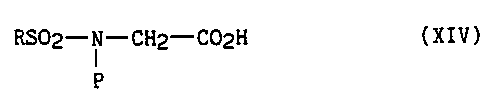 dans laquelle R a la même signification que dans la formule (I) et P représente un groupement protecteur classique de la fonction amine d'une amino-acide,
   pour conduire à l'aldéhyde de formule (XV) : 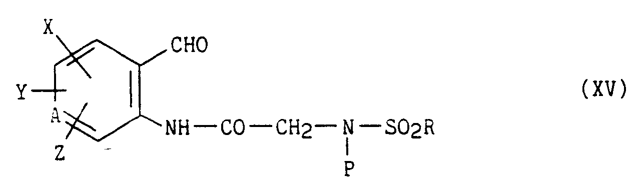 dans laquelle X, Y, Z, A, P et R ont la même signification que précédemment,
   qui est cyclisé, en milieu alcoolique, en présence d'un alcoolate de métal alcalin, d'une amine tertiaire ou d'un hydroxyde de métal alcalin,
   pour conduire à la quinoléinone de formule (XVI) : 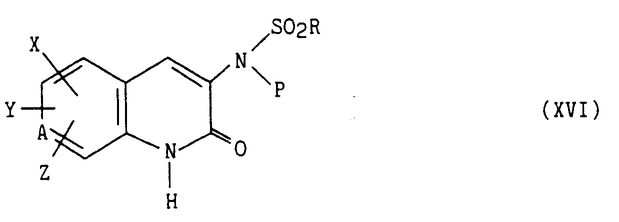 dans laquelle X, Y, Z, A, R et P ont la même signification que précédemment,
   qui est déprotégé en présence de chlorure d'aluminium dans un solvant inerte ou par hydrogénation catalytique,
   pour conduire à un composé de formule (I),
   composé de formule (I),
   - qui, lorsque X et/ou Y et/ou Z représentent un atome d'hydrogène, peut subir des substitutions électrophiles, selon des techniques classiques de substitution des noyaux aromatiques, conduisant à un composé de formule (I) mono, di ou trisubstitué sur le noyau phényl de la quinoléinone,
   - qui, lorsque X, Y ou Z représentent un groupement nitro, peut être transformé en dérivé amino ou acylamino correspondant, dérivé amino qui lui-même peut être transformé en dérivé azido correspondant selon des techniques classiques de la chimie organique,
   - qui, lorsque X représente un groupement nitro et Y, situé sur un carbone adjacent représente un groupement azido, peuvent subir une cyclisation pour conduire au dérivé 1,2,5-oxadiazolo correspondant,
   - qui peut être, le cas échéant, purifié selon une technique classique de purification,
   - dont on sépare, le cas échéant, les isomères selon une technique classique de séparation,
   - que l'on transforme, si on le souhaite, en ses sels d'addition à une base pharmaceutiquement acceptable.

Parmi les bases pharmaceutiquement acceptables, on peut citer à titre non limitatif l'hydroxyde de sodium, l'hydroxyde de potassium, la tertbutylamine, la diéthylamine, l'éthylènediamine...

Les composés de formule (I) possèdent des propriétés pharmacologiques très intéressantes.

Le double mécanisme d'action des composés de la présente invention a pu être étudié grâce aux techniques de binding membranaire et d' électrophysiologie.

### 1/ Binding membranaire

Il est réalisé à partir d'un homogénat de cortex de Rat. Le culot membranaire est classiquement préparé dans un tampon saccharose par centrifugations différentielles puis congelé jusqu'à utilisation. Après décongélation, 200 µl d'homogénat de membranes sont repris dans un tampon Tris HCl (30 mM), CaCl₂ (2,5 mM), pH 7,4 et incubés à 4°C pendant 30 minutes en présence de 25 µl de ³H-AMPA ou de ³H-Kainate et de 25 µl du produit à tester. La liaison non-spécifique est déterminée en présence de 10 µM de quisqualate (binding AMPA) ou de 10 µM d'acide kainique (binding kainate). Les membranes sont ensuite isolées par filtration. Après séchage des filtres, la radioactivité est mesurée par scintillation.

### 2/ Courants induits par les AAE dans l'oocyte de Xenopus

Des oocytes de Xenopus sont injectés de 50 ng d'ARNm poly (A⁺) isolés de cortex cérébral de Rat et incubés 2 à 3 Jours à 18°C pour en permettre l'expression protéique. Les courants entrants induits par les AAE sont mesurés dans un milieu de composition : NaCl (82,5 mM), KCl (2,5 mM), CaCl₂ (1 mM), MgCl₂ (1 mM), NaH₂PO₄ (1 mM), HEPES (5 mM), pH 7,4 par la méthode du Voltage-clamp à 2 électrodes (potentiel = - 60 mV). Pour la mesure des courants induits par le NMDA et la glycine, MgCl₂ est absent du milieu et le CaCl₂ est amené à la concentration de 2 mM. Les agonistes AAE induisant les courants sont utilisés aux concentrations suivantes : kainate : 100 µM ; AMPA : 30 µM ; glycine/NMDA : 3/30 µM. Le produit étudié est appliqué 30 secondes avant et durant l'application de l'agoniste.

### 3/ Potentiels excitateurs post-synaptiques (EPSP) hippocampiques induits par stimulation des voies afférentes excitatrices glutamatergiques (collatérales de Schäffer)

Des coupes transversales (500 µm) d'hippocampe de Rat sont placées dans un milieu de composition : NaCl (120 mM), KCl (5 mM), CaCl₂ (1 mM), MgSO₄ (1 mM), KH₂PO₄ (1,3 mM), NaHCO₃ (20 mM), HEPES (10 mM), glucose (10 mM), pH 7,35. Les "EPSP" sont enregistrés dans le champ dendritique de la région CA1 lors de stimulations toutes les 30 secondes de la stratum radiatum (50-100 µA, 20 µs). Les EPSP sont évalués au maximum de l'onde négative. Le produit étudié est appliqué durant 10 minutes.

Les composés de la présente invention ont été étudiés en comparaison aux plus récents composés décrits pour leur interaction avec le récepteur AMPA : ce sont des dérivés de quinoxaline-2,3-dione et plus particulièrement le 6-cyano-7-nitroquinoxaline-2,3-dione (CNQX décrit dans le brevet EP 260467) et le 6-nitro-7-sulfamoylbenzo[f]quinoxaline-2,3-dione (NBQX décrit dans le brevet EP 283959).

Les études de binding ont montré que les dérivés de l'invention se lient sur le récepteur AMPA avec une intensité très intéressante puisque le Ki est inférieur à 10⁻⁶M.

| | |
|---|---|
| CNQX | Ki = 0,07 µM |
| Exemple 2 | Ki = 0,6 µM |

De la même manière, ils sont capables d'inhiber l'activation fonctionnelle des courants par l'AMPA et le kainate dans l'oocyte de Xenopus.

| | | |
|---|---|---|
| Courant AMPA | CNQX | Ki = 0,40 µM |
| | NBQX | Ki = 0,06 µM |
| | Exemple 2 | Ki = 0,37 µM |

| | | |
|---|---|---|
| Courant Kainate | CNQX | IC50 = 0,27 µM |
| | NBQX | IC50 = 0,06 µM |
| | Exemple 2 | IC50 = 0,50 µM |

Il en est de même pour l'inhibition des EPSP hippocampiques induits par l'activation des récepteurs AMPA/Kainate (donc mesurés en présence de Mg⁺⁺ qui bloque les récepteurs NMDA).

| | |
|---|---|
| CNQX | IC50 = 2,9 µM |
| NBQX | IC50 = 1 µM |
| Exemple 2 | IC50 = 10 µM |

Bien que l'activité des composés de l'invention vis-à-vis des récepteurs AMPA/Kainate soit à 6 à 10 fois plus faible que celles des quinoxalinediones, leur originalité, donc leur intérêt thérapeutique, tient dans le fait qu'ils peuvent exercer une inhibition conjointe des phénomènes électrophysiologiques induits par l'activation des récepteurs NMDA/Glycine (donc mesurée en absence de magnésium).

| | | |
|---|---|---|
| Oocyte de Xenopus | CNQX | Ki = 2,1 µM |
| | NBQX | Ki > 1000 µM |
| | Exemple 2 | Ki = 0,62 µM |

| | | |
|---|---|---|
| EPSP hippocampique | CNQX | IC50 = 100 µM |
| | NBQX | IC50 > 100 µM |
| | Exemple 2 | IC50 = 40 µM |

Ce double mécanisme d'action peut être apprécié en calculant les rapports entre les activités inhibitrices vis-à-vis du récepteur AMPA et du récepteur NMDA/Glycine.

| | Oocyte de Xenopus Ki AMPA/Ki NMDA/Gly | EPSP hippocampique IC50 AMPA-Kainate/IC50 NMDA-Glycine |
|---|---|---|
| CNQX | 0,19 | 0,03 |
| NBQX | << 0,001 | << 0,001 |
| Exemple 2 | 0,60 | 0,25 |

Ainsi, le double mécanisme des composés de l'invention est 3 à 8 fois plus marqué que dans le cas de la quinoxalinedione CNQX, ce qui confère une potentialité thérapeutique bien plus importante vis-à-vis des phénomènes neurotoxiques liés aux AAE.

La présente invention a également pour objet les compositions pharmaceutiques renfermant comme principe actif au moins un composé de formule (I), seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques.

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 1 et 1000 mg pour un traitement en 1 à 3 prises par 24 heures.

Les exemples suivants illustrent l'invention et ne la limitent en aucune façon.

Les produits de départ utilisés sont des produits connus où préparés selon des modes opératoires connus.

### EXEMPLE 1 : 3-(Trifluorométhylsulfonylamino)-2-(1H)-quinoléinone

### Stade A : 2-Méthoxy-3-nitroquinoléine

7,9 mmoles de 3-nitro-2-(1H)quinoléinone (préparé à partir d'acide anthranilique selon la technique décrite par C.T. ALABASTER et coll., dans J. Med. Chem., 31, 2048-2056, 1988) dissoutes dans 10 ml d'oxychlorure de phosphore sont portées 2 heures à reflux. L'excès d'oxychlorure de phosphore est évaporé sous pression réduite. L'huile résiduelle est dissoute dans 50 ml de dichlorométhane. Cette phase organique est lavée à l'eau jusqu'à pH neutre, séchée et évaporée sous vide. Après dissolution du résidu dans 40 ml de méthanol, on ajoute, sous agitation, 12 ml d'une solution 1N de méthylate de sodium dans le méthanol. L'ensemble est porté à reflux 15 minutes. Après refroidissement et évaporation du méthanol, le résidu est repris par 50 ml de dichlorométhane et 50 ml d'eau. La phase organique est décantée, séchée et évaporée sous vide. Le produit attendu est obtenu après purification par chromatographie sur gel de silice en utilisant comme solvant d'élution un mélange hexane/acétate d'éthyle (9/1).
Point de fusion : 105°C

### Stade B : 2-Méthoxy-3-aminoquinoléine

7,8 mmoles du produit obtenu au stade précédent sont mises en suspension dans 50 ml d'éthanol. 39 mmoles de chlorure d'étain dihydraté sont ajoutées à cette suspension et l'ensemble est porté à reflux une heure. A la solution ainsi obtenue, 400 ml de chloroforme et 100 ml d'eau sont ajoutés. La solution est basifiée par addition d'une solution aqueuse de bicarbonate de sodium. La suspension qui se forme est filtrée, la phase organique est décantée et séparée de la phase aqueuse qui est à nouveau extraite par du chloroforme. Les phases organiques réunies sont séchées et évaporées. Le produit attendu est obtenu après purification du résidu par chromatographie sur gel de silice en utilisant comme solvant d'élution un mélange hexane/acétate d'éthyle (9/1).
Point de fusion : 84°C

### Stade C : 2-Méthoxy-3-(trifluorométhylsulfonylamino)quinoléine

3,2 mmoles du produit obtenu au stade précédent sont dissoutes dans 50 ml de dichlorométhane à 0°C. 3,3 mmoles de triéthylamine sont alors ajoutées à cette solution puis, goutte à goutte, 4 mmoles d'anhydride trifluorométhanesulfonique. L'ensemble est agité 30 minutes à 0°C puis 30 minutes à température ambiante. Après lavage par 10 ml d'acide chlorhydrique 1N, la phase organique est extraite par une solution aqueuse 1N d'hydroxyde de potassium puis par de l'eau. Les phases aqueuses réunies sont acidifiées par de l'acide chlorhydrique 1N et extraites par du dichlorométhane. La phase organique ainsi obtenue est alors séchée et évaporée. Le produit attendu est obtenu sous forme d'une poudre blanche.
Point de fusion : 135°C

### Stade D : 3-(Trifluorométhylsulfonylamino)-2-(1H)quinoléinone

3,4 mmoles du produit obtenu au stade précédent dissoutes dans 20 ml de méthanol et 10 ml d'acide chlorhydrique concentré sont portées 2 heures à reflux. L'ensemble est abandonné une nuit à température ambiante. Le produit attendu est obtenu après filtration et séchage du solide blanc formé.
Point de fusion : 204°C

| Microanalyse élémentaire : | | | | |
|---|---|---|---|---|
| | C % | H % | N % | S % |
| calculé | 41,10 | 2,41 | 9,59 | 10,97 |
| trouvé | 41,25 | 2,67 | 9,73 | 11,28 |

### EXEMPLE 2 : 7-Nitro-3-(trifluorométhylsulfonylamino)-2-(1H)-quinoléinone

### Stade A : 3-Nitro-6-hydroxyméthylaniline

55 mmoles d'acide 5-nitroanthranilique sont dissoutes dans 250 ml de tétrahydrofurane anhydre. 110 mmoles du complexe boranediméthylsulfure sont ajoutées, goutte à goutte, à la solution précédente. La solution est alors portée 2 heures à reflux, refroidie dans de la glace et additionnée, goutte à goutte, de 10 ml d'eau. A la fin du dégagement gazeux, 30 ml d'acide chlorhydrique concentré sont ajoutés à l'ensemble précédent et la solution est portée 30 minutes à reflux. Après refroidissement, elle est versée sur 500 ml de glace. La phase aqueuse est lavée par 50 ml de dichlorométhane, alcalinisée par une solution aqueuse concentrée d'hydroxyde de sodium (30 %) et extraite plusieurs fois par du dichlorométhane. Les phases organiques sont réunies, séchées et évaporées sous vide et conduisent au produit attendu.
Point de fusion : 130°C

### Stade B : 3-Nitro-6-formylaniline

A une solution contenant 37,6 mmoles du produit obtenu au stade précédent dans le dichlorométhane, 75 mmoles d'oxyde de manganèse sont ajoutées en une fois et la suspension ainsi obtenue est agitée 2 heures, à température ambiante. Après filtration, le filtrat est évaporé et le produit attendu est obtenu après purification du résidu par chromatographie sur gel de silice en utilisant comme solvant d'élution un mélange hexane/acétate d'éthyle (75/25).
Point de fusion : 123°C

### Stade C : Ester méthylique de l'acide 7-nitro-2-(1H)-quinoléinone-3-carboxylique

A une suspension contenant 12 mmoles du produit obtenu au stade B dans 25 ml de méthanol, on ajoute 36 mmoles de diméthylmalonate et 24 ml d'une solution 1N de méthylate de sodium dans le méthanol. La suspension est agitée deux jours à température ambiante. Le précipité vert formé est alors filtré, lavé par du méthanol puis par de l'éther, séché et fournit le produit attendu, utilisé tel quel au stade D.

### Stade D : Ester méthylique de l'acide 7-nitro-2-méthoxyquinoléine-3-carboxylique

Le produit attendu est obtenu selon le même mode opératoire que celui décrit au stade A de l'exemple 1, mais le produit obtenu au stade C, dissous dans 10 ml d'oxychlorure de phosphore, est porté 6 heures à reflux.
Point de fusion : 174°C

### Stade E : Acide 2-méthoxy-7-nitroquinoléine-3-carboxylique

75 mmoles du produit obtenu au stade précédent sont hydrolysées, en présence de 20 ml d'une solution 1N d'hydroxyde de potassium et de 80 ml de méthanol, pendant 1 heure 30 à reflux. Après évaporation du méthanol, la suspension est diluée à l'eau. La phase aqueuse, lavée par du dichlorométhane, est acidifiée par de l'acide chlorhydrique 1N et le solide blanc qui se forme est extrait par du dichlorométhane. La phase organique, séchée et évaporée, conduit au produit attendu sous forme de solide blanc.
Point de fusion : 203°C

### Stade F : 3-Tertbutoxycarbonylamino-7-nitro-2-méthoxyquinoléine

6 mmoles du produit obtenu au stade précédent, 6,5 mmoles de triéthylamine, 6,5 mmoles d'azoture de diphénoxyphosphoryl sont mélangées dans 100 ml de toluène. Cette solution est chauffée sous atmosphère d'azote à 75°C jusqu'à la fin du dégagement gazeux. 100 ml de tertbutanol anhydre sont ajoutés et l'ensemble est porté une heure à reflux. Après refroidissement de la solution, le solide jaune est filtré et lavé par du dichlorométhane. Les solvants sont alors réunis et évaporés. Le résidu est repris par 100 ml d'eau et 100 ml de dichlorométhane. La phase organique est séchée et évaporée. Le produit attendu est obtenu après purification du résidu par chromatographie sur gel de silice en utilisant comme solvant d'élution un mélange hexane/acétate d'éthyle (9/1).
Point de fusion : 167°C

### Stade G : 3-Amino-2-méthoxy-7-nitroquinoléine

Une suspension contenant 1,5 mmoles du produit obtenu au stade précédent dans 25 ml d'acétate d'éthyle anhydre est refroidie à 0°C et saturée par de l'acide chlorhydrique gazeux sec. Après 3 heures d'agitation, le solvant est évaporé sous vide, à température ambiante, en milieu anhydre. 25 ml d'une solution 1N d'hydroxyde de sodium sont alors ajoutés et la phase aqueuse est extraite par de l'acétate d'éthyle. La phase organique est séchée et évaporée. Le produit attendu est obtenu, après purification du solide jaune ainsi obtenu par dissolution dans du dichlorométhane, filtration et évaporation.
Point de fusion : 166°C

### Stade H : 7-Nitro-3-(trifluorométhylsulfonylamino)-2-(1H)-quinoléinone

A une solution, à 0°C, contenant 1,7 mmoles du composé obtenu au stade précédent et 2 mmoles de triéthylamine dans 50 ml de dichlorométhane, on ajoute, goutte à goutte, 2 mmoles d'anhydride trifluorométhanesulfonique. La solution, après retour à température ambiante, est agitée une nuit, lavée à l'eau, séchée et évaporée. Le résidu est repris par 2 ml de méthanol et 2 ml d'acide chlorhydrique concentré. Cette solution est portée 2 heures à reflux, refroidie, extraite par de l'acétate d'éthyle. La phase organique est alors séchée et évaporée. Le produit attendu est alors obtenu après purification du résidu sur gel de silice en utilisant comme solvant d'élution un mélange dichlorométhane/éthanol/acide acétique (95/4,5/0,5). Il est alors cristallisé dans un mélange toluène/acétate d'éthyle (9/1) et séché.
Point de fusion : 240°C

| Microanalyse élémentaire : | | | | |
|---|---|---|---|---|
| | C % | H % | N % | S % |
| calculé | 35,62 | 1,79 | 12,46 | 9,50 |
| trouvé | 35,76 | 2,00 | 12,39 | 9,56 |

### EXEMPLE 2 Bis : 7-Nitro-3-(trifluorométhylsulfonylamino)-2-(1H)-quinoléinone

Les stades A et B de ce procédé sont identiques à ceux décrits dans l'exemple 2. En revanche, les stades C à H de l'exemple 2 peuvent être remplacés par les stades C à E suivants et conduire au même composé.

### Stade C : N-[2-(N'-Benzyl-N'-trifluorométhylsulfonylamino)acétyl]-3-nitro-6-formylaniline

A une solution, à 0°C, contenant 16,5 mmoles du composé décrit au stade précédent dans 100 ml d'acétone, sont additionnées, goutte à goutte, 8,5 mmoles du chlorure de l'acide 2-[(N-benzyl-N-trifluorométhylsulfonyl)amino]acétique. La solution est maintenue 18 heures sous agitation, à température ambiante, le solvant est évaporé et le résidu repris par 100 ml d'acétate d'éthyle. La phase organique est lavée par de l'acide chlorhydrique 1N puis par une solution d'hydroxyde de sodium 1N, enfin par de l'eau puis séchée et évaporée. Le produit attendu est obtenu après purification du résidu par chromatographie sur gel de silice en utilisant comme solvant d'élution un mélange hexane/acétate d'éthyle (8/2).
Point de fusion : 157°C

### Stade D : 3-[(N-Benzyl-N-trifluorométhylsulfonyl)amino]-7-nitro-2-(1H)-quinoléinone

Une solution contenant 1,8 mmoles du composé obtenu au stade précédent dans 20 ml de méthanol et 2 ml d'une solution 1N de méthylate de sodium dans le méthanol est agitée 2 jours à température ambiante. Cette solution est versée sur 100 ml de glace et extraite plusieurs fois par de l'acétate d'éthyle. Les phases organiques réunies sont séchées et évaporées. Le produit attendu est obtenu après purification du résidu par chromatographie sur gel de silice en utilisant comme solvant d'élution un mélange hexane/acétate d'éthyle (75/25).
Point de fusion : 224°C

### Stade E : 7-Nitro-3-(trifluorométhylsulfonylamino)-2-(1H)-quinoléinone

A une solution contenant 1,2 mmoles du produit obtenu au stade précédent dans 20 ml de dichlorométhane, à température ambiante, sont ajoutées 6 mmoles de chlorure d'aluminium. L'ensemble est agité 2 heures puis versé sur 50 ml de glace additionnée de 20 ml d'acide chlorhydrique 1N. Après extraction de la phase aqueuse par de l'acétate d'éthyle, la phase organique est séchée et évaporée. Le produit attendu est obtenu après purification par chromatographie sur gel de silice en utilisant comme solvant d'élution un mélange dichlorométhane/éthanol/acide acétique (95/4,5/0,5). Il est cristallisé dans un mélange toluène/acétate d'éthyle (9/1) et séché sous vide à 75°C.
Point de fusion : 240°C

| Microanalyse élémentaire : | | | | |
|---|---|---|---|---|
| | C % | H % | N % | S % |
| calculé | 35,62 | 1,79 | 12,46 | 9,50 |
| trouvé | 35,21 | 1,95 | 12,24 | 9,55 |

Les exemples 3 à 5 et 8 à 10 ont été obtenus en procédant selon l'un des procédés décrits dans les exemples 1, 2 ou 2 bis en utilisant les matières premières correspondantes :

### EXEMPLE 3 : 3-Méthylsulfonylamino-2-(1H)-quinoléinone

Ce composé a été obtenu selon le procédé décrit dans l'exemple 1.
Point de fusion : 252°C

| Microanalyse élémentaire : | | | | |
|---|---|---|---|---|
| | C % | H % | N % | S % |
| calculé | 50,41 | 4,23 | 11,76 | 13,46 |
| trouvé | 50,38 | 4,35 | 11,60 | 13,34 |

### EXEMPLE 4 : 3-(Benzènesulfonylamino)-2-(1H)-quinoléinone

Ce composé a été obtenu selon le procédé décrit dans l'exemple 1.
Point de fusion : 235°C

| Microanalyse élémentaire : | | | | |
|---|---|---|---|---|
| | C % | H % | N % | S % |
| calculé | 59,99 | 4,03 | 9,33 | 10,68 |
| trouvé | 59,78 | 4,08 | 9,30 | 10,19 |

### EXEMPLE 5 : 3-(2-Thiénylsulfonylamino)-2-(1H)-quinoléinone

Ce composé a été obtenu selon le procédé décrit dans l'exemple 1.
Point de fusion : 240°C

| Microanalyse élémentaire : | | | | |
|---|---|---|---|---|
| | C % | H % | N % | S % |
| calculé | 50,97 | 3,29 | 9,14 | 20,93 |
| trouvé | 51,06 | 3,25 | 9,03 | 20,55 |

### EXEMPLE 6 : 8-Nitro-3-(trifluorométhylsulfonylamino)-2-(1H)-quinoléinone

et

### EXEMPLE 7 : 6-Nitro-3-(trifluorométhylsulfonylamino)-2-(1H)-quinoléinone

Ces deux composés ont été obtenus par nitration électrophile du composé décrit dans l'exemple 1.

Une solution contenant 2 ml d'acide nitrique concentré et 1 ml d'acide sulfurique concentré est préparée à 0°C. 330 µl de cette solution sont ajoutés goutte à goutte, à 0°C, à une solution contenant 3,4 mmoles du composé décrit dans l'exemple 1 dans 2 ml d'acide sulfurique concentré. L'ensemble est alors agité 10 minutes à 0°C puis 1 heure 30 minutes à température ambiante. Après refroidissement du milieu réactionnel à 0°C, 8 ml d'eau sont additionnés, goutte à goutte, sous agitation. Le précipité jaune formé est filtré, rincé à l'eau et séché. Les deux isomères sont séparés par chromatographie sur gel de silice en utilisant comme solvant d'élution un mélange dichlorométhane/éthanol/acide acétique (90/9/1).

### EXEMPLE 6 :

L'isomère 8-nitro est élué le premier et est recristallisé dans un mélange acétate d'éthyle/hexane.
Point de fusion : 205°C

| Microanalyse élémentaire : | | | | |
|---|---|---|---|---|
| | C % | H % | N % | S % |
| calculé | 35,62 | 1,79 | 12,46 | 9,51 |
| trouvé | 35,82 | 2,10 | 12,44 | 9,72 |

### EXEMPLE 7 :

L'isomère 6-nitro est ensuite élué et est recristallisé dans de l'isopropanol.
Point de fusion : > 260°C

| Microanalyse élémentaire : | | | | |
|---|---|---|---|---|
| | C % | H % | N % | S % |
| calculé | 35,62 | 1,79 | 12,46 | 9,51 |
| trouvé | 35,88 | 2,10 | 12,45 | 9,81 |

### EXEMPLE 8 : 6,7-Méthylènedioxy-3-(trifluorométhylsulfonylamino)-2-(1H)-quinoléinone

Ce composé a été obtenu selon le procédé décrit dans l'exemple 1.
Point de fusion : > 250°C

| Microanalyse élémentaire : | | | | |
|---|---|---|---|---|
| | C % | H % | N % | S % |
| calculé | 39,29 | 2,10 | 8,33 | 9,54 |
| trouvé | 39,44 | 2,22 | 8,32 | 9,84 |

### EXEMPLE 9 : 6,8-Dichloro-3-(trifluorométhylsulfonylamino)-2-(1H)-quinoléinone

Ce composé a été obtenu selon le procédé décrit dans l'exemple 2 bis. L'intermédiaire obtenu au stade C n'est pas isolé mais se cyclise spontanément dans les conditions de la réaction pour conduire à l'intermédiaire obtenu au stade D.
Point de fusion : 215°C

| Microanalyse élémentaire : | | | | | |
|---|---|---|---|---|---|
| | C % | H % | N % | Cl % | S % |
| calculé | 33,26 | 1,40 | 7,76 | 19,63 | 8,88 |
| trouvé | 33,47 | 1,70 | 7,74 | 19,65 | 9,05 |

### EXEMPLE 10 : 7-Chloro-3-(trifluorométhylsulfonylamino)-2-(1H)-quinoléinone

Ce composé a été obtenu selon le procédé décrit dans l'exemple 2 bis.
Point de fusion : 250°C

| Microanalyse élémentaire : | | | | | |
|---|---|---|---|---|---|
| | C % | H % | N % | S % | Cl % |
| calculé | 36,77 | 1,85 | 8,58 | 9,82 | 10,85 |
| trouvé | 36,82 | 2,10 | 8,53 | 9,80 | 11,02 |

### EXEMPLE 11 : 7-Chloro-3-(méthylsulfonylamino)-2-(1H)-quinoléinone

Ce composé a été obtenu selon le procédé décrit dans l'exemple 2 bis.
Point de fusion : > 260°C

| Microanalyse élémentaire : | | | | |
|---|---|---|---|---|
| | C % | H % | N % | S % |
| calculé | 44,04 | 3,33 | 10,27 | 11,76 |
| trouvé | 44,07 | 3,45 | 10,03 | 11,61 |

### EXEMPLE 12 : 6-Bromo-3-(trifluorométhylsulfonylamino)-2-(1H)-quinoléinone

A une suspension contenant 3,4 mmoles du composé décrit dans l'exemple 1, dans 10 ml d'acide acétique, est ajoutée, goutte à goutte, à température ambiante, une solution de 300 µl de brome dans 10 ml d'acide acétique glacial. Après une nuit d'agitation à température ambiante, 10 ml d'eau sont ajoutés. Le produit attendu est obtenu par filtration du précipité et est purifié par chromatographie sur gel de silice en utilisant comme solvant d'élution un mélange dichlorométhane/éthanol/acide acétique (90/9,5/0,5).
Point de fusion : > 260°C

| Microanalyse élémentaire : | | | | | |
|---|---|---|---|---|---|
| | C % | H % | N % | Br | S % |
| calculé | 32,36 | 1,63 | 7,55 | 21,53 | 8,64 |
| trouvé | 36,52 | 1,93 | 7,60 | 21,81 | 8,28 |

### EXEMPLE 13 : 6-Acétamido-3-(trifluorométhylsulfonylamino)-2-(1H)-quinoléinone

Une suspension contenant 1,48 mmoles du composé décrit dans l'exemple 7 dans 100 ml d'éthanol est hydrogénée pendant une heure à pression atmosphérique en présence de 50 mg de PtO₂. On évapore l'éthanol sous vide, on dissout le résidu dans 100 ml de THF et le catalyseur est filtré. Au filtrat on ajoute 1,63 mmoles de triéthylamine puis 1,48 mmoles d'anhydride acétique et on agite la solution réactionnelle à température ambiante pendant 1 h 30. Le THF est évaporé sous vide et on reprend le résidu dans de l'acétate d'éthyle. On lave la phase organique avec une solution d'HCl 1N puis on extrait avec une solution de NaOH 1N. La phase aqueuse basique est réacidifiée avec HCl 1N et extraite avec de l'acétate d'éthyle. Après séchage et purification par chromatographie sur silice (dichlorométhane/éthanol/acide acétique : 90/9/1), le produit attendu est recristallisé dans de l'acétate d'éthyle.
Point de fusion : > 260°C

| Microanalyse élémentaire : | | | | |
|---|---|---|---|---|
| | C % | H % | N % | S % |
| calculé | 41,26 | 2,89 | 12,03 | 9,18 |
| trouvé | 41,05 | 3,16 | 11,83 | 9,44 |

### EXEMPLE 14 : 6,7-Dinitro-3-(trifluorométhylsulfonylamino)-2-(1H)-quinoléinone

2,96 mmoles du composé décrit dans l'exemple 2 sont dissoutes dans 2 ml d'acide sulfurique à 96 % et l'ensemble est refroidi à 0°C. On prépare à 0°C une solution de 0,75 ml d'acide sulfurique 96 % et 1 ml d'acide nitrique 86 %. On ajoute goutte à goutte 275 µl de cette solution à la solution réactionnelle et on agite 15 mn à 0°C pùis 1 h en laissant revenir à température ambiante. On ajoute 5 ml d'eau glacée et on extrait avec de l'acétate d'éthyle. Après séchage, le produit attendu est purifié par chromatographie sur silice (dichlorométhane/éthanol/acide acétique : 90/9/1).
Point de fusion : 236°C

| Microanalyse élémentaire : | | | | |
|---|---|---|---|---|
| | C % | H % | N % | S % |
| calculé | 31,42 | 1,32 | 14,66 | 8,39 |
| trouvé | 31,38 | 1,51 | 13,95 | 8,12 |

### EXEMPLE 15 : 7-Amino-3-(trifluorométhylsulfonylamino)-2-(1H)-quinoléinone

On agite pendant 1 h à reflux 1,47 mmoles du composé décrit dans l'exemple 2 et 6 mmoles de chlorure stanneux dihydraté dans 50 ml d'éthanol. On extrait avec HCl 3H (3x30 ml). Les phases aqueuses sont rassemblées et neutralisées avec une solution de NaOH 3N jusqu'à PH 5-6. On extrait avec de l'acétate d'éthyle (2x50 ml), sèche et recristallise dans un mélange acétate d'éthyle/hexane.
Point de fusion : > 260°C

| Microanalyse élémentaire : | | | | |
|---|---|---|---|---|
| | C % | H % | N % | S % |
| calculé | 39,09 | 2,62 | 13,68 | 10,44 |
| trouvé | 39,18 | 3,08 | 13,20 | 10,28 |

### EXEMPLE 16 : 7-Acétamido-3-(trifluorométhylsulfonylamino)-2-(1H)-quinoléinone

A une solution contenant 1 mmole du composé de l'exemple 15 dans 100 ml de THF, on ajoute 1,1 mmoles de triéthylamine et 1,1 mmoles d'anhydride acétique. On agite une nuit à reflux et on évapore le THF sous vide. Le résidu est repris dans de l'acétate d'éthyle. On lave la phase organique avec une solution de HCl 1N et on extrait avec une solution de NaOH 1N. La phase aqueuse basique est lavée avec de l'acétate d'éthyle et est acidifiée avec une solution de HCl 1N pour donner un précipité blanc qui est filtré et recristallisé dans de l'éthanol.
Point de fusion : 315°C

| Microanalyse élémentaire : | | | | |
|---|---|---|---|---|
| | C % | H % | N % | S % |
| calculé | 41,26 | 2,89 | 12,03 | 9,18 |
| trouvé | 41,31 | 3,30 | 11,82 | 9,23 |

### EXEMPLE 17 : 7-Azido-3-(trifluorométhylsulfonylamino)-2-(1H)quinoléinone

On agite pendant 10 mn à 0°C une suspension de 1,3 mmoles du composé de l'exemple 15 dans 6 ml d'une solution aqueuse 48 % d'acide tétrafluoroborique. On ajoute 10 ml d'eau puis goutte à goutte une solution de 1,95 mmoles de nitrite de sodium dans 3 ml d'eau. L'ensemble est agité 30 minutes à 0°C et on ajoute goutte à goutte une solution de 1,95 mmoles d'azoture de sodium dans 3 ml d'eau. Après 10 minutes d'agitation à 0°C, on laisse revenir à température ambiante en agitant pendant 3 h. On ajoute 20 ml d'eau et on extrait avec de l'acétate d'éthyle (2x20 ml). La phase organique est ensuite lavée avec une solution de HCl 1N et séchée sur MgSO₄. On concentre la phase organique jusqu'à la formation d'un précipité, on filtre et recristallise le précipité dans de l'acétate d'éthyle.
Point de fusion : 235°C

| Microanalyse élémentaire : | | | | |
|---|---|---|---|---|
| | C % | H % | N % | S % |
| calculé | 36,04 | 1,81 | 21,02 | 9,62 |
| trouvé | 36,18 | 2,09 | 20,66 | 10,02 |

### EXEMPLE 18 : 5,7-Dichloro-3-(trifluorométhylsulfonylamino)-2-(1H)-quinoléinone

Ce composé a été obtenu selon le procédé décrit dans l'exemple 2 bis.
Point de fusion : 254°C

| Microanalyse élémentaire : | | | | | |
|---|---|---|---|---|---|
| | C % | H % | N % | Cl % | S % |
| calculé | 33,26 | 1,40 | 7,76 | 19,63 | 8,88 |
| trouvé | 33,11 | 1,73 | 7,78 | 19,29 | 8,83 |

### EXEMPLE 19 : 6,7-Dichloro-3-(trifluorométhylsulfonylamino)-2-(1H)-quinoléinone

Ce composé a été obtenu selon le procédé décrit dans l'exemple 2 bis.
Point de fusion : 289°C

| Microanalyse élémentaire : | | | | | |
|---|---|---|---|---|---|
| | C % | H % | N % | Cl % | S % |
| calculé | 33,26 | 1,40 | 7,76 | 19,63 | 8,88 |
| trouvé | 33,47 | 1,84 | 7,91 | 19,42 | 9,01 |

### EXEMPLE 20 : 7-Aza-3-(trifluorométhylsulfonylamino)-2-(1H)-quinoléinone, sel de sodium

Le composé a été obtenu du stade A au stade D selon le procédé décrit dans l'exemple 2 bis. L'intermédiaire obtenu au stade C n'est pas isolé mais se cyclise spontanément dans les conditions de la réaction pour conduire à l'intermédiaire obtenu au stade D.

### Stade E : 7-Aza-3-(trifluorométhylsulfonylamino)-2-(1H)-quinoléinone, sel de sodium

On hydrogène pendant 24 h à pression atmosphérique dans 200 ml d'éthanol 0,6 mmoles de 7-aza-3-[(N-benzyl-N-trifluorométhylsulfonyl) amino]-2-(1H)-quinoléinone en présence de 20 mg de PtO₂. Le précipité qui s'est formé est dissous en ajoutant 10 ml d'éthanol chlorhydrique 2N et en chauffant à environ 50°C. On filtre le catalyseur et on évapore à sec. Le résidu blanc est séché sous vide 1 nuit. On le met en suspension dans 20 ml d'eau distillée et on ajoute 0,6 mmoles d'une solution aqueuse de NaOH 1N. On agite la solution pendant 15 min, filtre les petits insolubles et on lyophilise.

| Microanalyse élémentaire : | | | | |
|---|---|---|---|---|
| | C % | H % | N % | S % |
| calculé | 31,77 | 1,65 | 12,35 | 9,42 |
| trouvé | 31,76 | 1,51 | 12,05 | 9,60 |

### EXEMPLE 21 : 7-(Trifluorométhylsulfonylamino)-6-hydroxy-(1,2,5-oxadiazolo [3,4-g]quinoléine, sel de sodium

### Stade A : 7-Azido-6-nitro-3-(trifluorométhylsulfonylamino)-2-(1H)-quinoléinone

On ajoute par petites portions 1,38 mmoles du composé de l'exemple 17 dans 3,5 ml d'acide nitrique 86 5 à 0°C. On agite 15 mn à 0°C et on verse sur de la glace. Le précipité jaune est filtré, rincé avec de l'eau et séché sous vide.

### Stade B : 7-(Trifluorométhylsulfonylamino)-6-hydroxy-(1,2,5-oxadiazolo)-[3,4-g]-quinoléine-1-oxyde

On agite à 100°C pendant 3 h une suspension contenant 0,7 mmoles du produit obtenu au stade précédent dans 50 ml de toluène. On évapore le toluène sous pression réduite, le résidu est repris dans de l'éther et le précipité est filtré.

### Stade C : 7-(Trifluorométhylsulfonylamino)-6-hydroxy-(1,2,5-oxadiazolo[3,4-g]quinoléine, sel de sodium

A une solution contenant 0,67 mmoles du produit obtenu au stade précédent dans 50 ml d'acétone, on ajoute 0,7 mmoles de triphénylphosphine. On agite 15 mn à température ambiante et on évapore à sec. On reprend le résidu dans 20 ml d'éther et on extrait avec NaOH 1N (2x10 ml). La phase aqueuse est acidifiée avec HCl 1N et on extrait avec de l'acétate d'éthyle. Après séchage, le produit attendu est obtenu par chromatographie sur silice (acétate d'éthyle/méthanol 95/5).
Point de fusion : 236°C

| Microanalyse élémentaire : | | | | |
|---|---|---|---|---|
| | C % | H % | N % | S % |
| calculé | 33,72 | 1,13 | 15,73 | 9,00 |
| trouvé | 33,37 | 1,46 | 15,17 | 9,44 |

### EXEMPLE 22 : Composition pharmaceutique

Formule de préparation pour 1000 comprimés dosés à 10 mg

| | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications (Revendications pour l'(les) Etat(s) contractant(s) suivant(s): AT, BE, CH, LI, DE, DK, FR, GB, GR, IE, IT, LU, NL, PT, SE)

1. Composés de formule (I) : 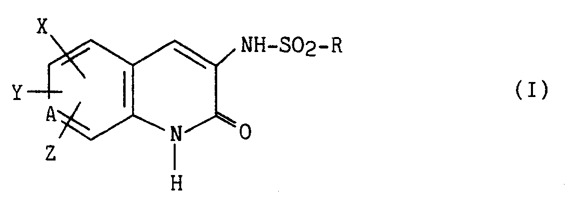 dans laquelle :
X, Y, Z :
- identiques ou différents, représentent un atome d'hydrogène, un atome d'halogène, un groupement nitro, cyano, azido, trihalogénométhyle, alkyle (C₁-C₆) linéaire ou ramifié, alkoxy (C₁-C₆) linéaire ou ramifié, amino (substitué ou non par un groupement acyl (C₁-C₆) linéaire ou ramifié),
- ou bien, lorsqu'ils sont situés sur deux carbones adjacents, forment avec les atomes de carbone auxquels ils sont attachés, un cycle phényle ou un hétérocycle à 5 ou 6 chaînons comportant de 1 à 3 hétéroatomes,
R représente un groupement alkyle (C₁-C₆) linéaire ou ramifié, trihalogénométhyle, phényle (substitué ou non par un ou plusieurs atomes d'halogène ou groupements alkyle (C₂-C₆) linéaire ou ramifié, trihalogénométhyle, alkoxy (C₁-C₆) linéaire ou ramifié), 2-thiényle (substitué ou non par un atome d'halogène), naphtyle ou styryle,
A représente un radical CH ou un atome d'azote,
leurs énantiomères, diastéréoisomères et épimères ainsi que leurs sels d'addition à une base pharmaceutiquement acceptable.

2. Composés de formule (I) selon la revendication 1 tels que R représente un groupement trihalogénométhyle.

3. Composés de formule (I) selon la revendication 1 tels que l'un au moins des groupements X, Y ou Z représente un groupement nitro.

4. Composés de formule (I) selon la revendication 1 tels que l'un au moins des groupements X, Y ou Z représente un groupement chloro.

5. Composés de formule (I) selon la revendication 1 tels que X, Y ou Z lorsqu'ils sont situés sur deux carbones adjacents forment avec les atomes de carbone auxquels ils sont attachés un cycle 1,2,5-oxadiazole.

6. Composé de formule (I) selon la revendication 1 qui est le 7-nitro-3(trifluorométhylsulfonylamino)-2-(1H)-quinoléinone.

7. Procédé de préparation des composés de formule (I) caractérisé en ce que l'on utilise comme produit de départ un composé de formule (II) : 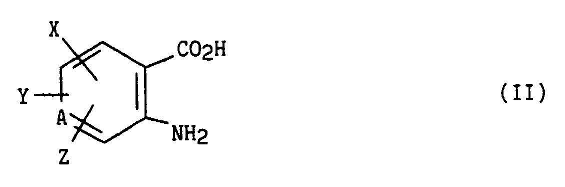 dans laquelle X, Y, Z et A ont la même signification que dans la formule (I),
que l'on réduit selon la nature des substituants X, Y et Z,
soit en milieu aprotique en présence d'hydrure de lithium aluminium, d'hydrure d'aluminium, de diborane ou des complexes de borane,
soit en milieu protique acide lorsque l'on utilise le cyanoborohydrure de sodium,
pour conduire à un alcool de formule (III) : 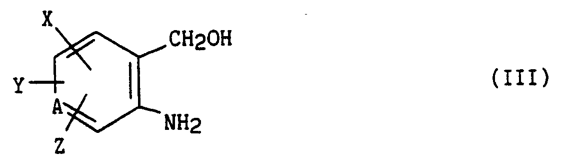 dans laquelle X, Y, Z et A ont la même signification que dans la formule (I),
que l'on oxyde à l'aide d'un oxyde métallique dans un solvant inerte, d'un halogénate de métal alcalin dans un solvant protique ou d'un chlorure d'acide dans le diméthylsulfoxyde,
pour conduire à l'adéhyde de formule (IV) : 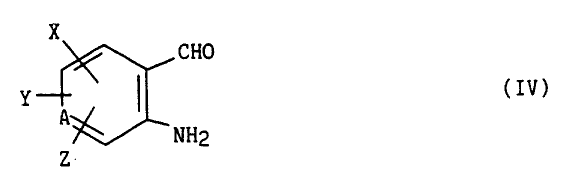 dans laquelle X, Y, Z et A ont la même signification que dans la formule (I),
qui est alors condensé :
1°/ 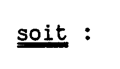 avec un malonate d'alkyle de formule (V), en milieu protique, en présence d'un alcoolate de métal alcalin, d'une amine tertiaire ou d'un hydroxyde de métal alcalin : 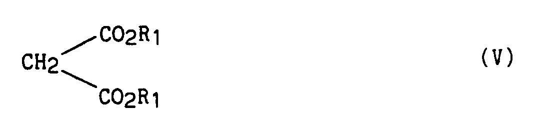 dans laquelle R₁ représente un groupement alkyle (C₁-C₆) linéaire ou ramifié, substitué ou non par un ou plusieurs groupements phényle,
pour conduire à une 2-(1H)-quinoléinone de formule (VI) : 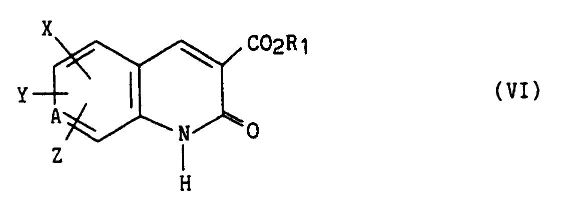 dans laquelle X, Y, Z, A et R₁ ont la même signification que précédemment, qui est protégée :
- par réaction avec un tétrafluoroborate de trialkyloxonium dans de l'éther ou le dichlorométhane,
- ou par réaction, dans un solvant inerte ou sans, d'un oxyhalogénure de phosphore, d'un pentahalogénure de phosphore, d'un halogénure de thionyle, de phosgène, de thiophosgène, de phosgène imminium ou de trimère de phosgène, suivie d'une réaction de la 2-halogénoquinoléine ainsi formée avec un alkoxyde de métal alcalin de formule R'₁OM (dans laquelle R'₁ a la même signification que R₁ et M représente un métal alcalin), en milieu alcoolique,
- ou par réaction avec l'hydrure de sodium, le butyllithium, un alcoolate de métal alcalin en présence d'un agent d'alkylation de formule R'₁V (dans laquelle R'₁ a la même signification que précédemment et V est un groupe partant),
pour conduire à la 2-alkoxyquinoléine de formule (VII) : 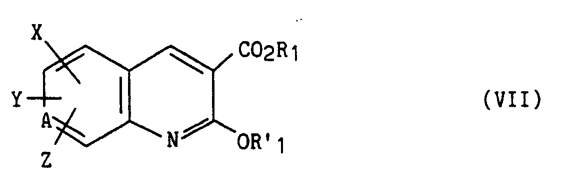 dans laquelle X, Y, Z, A, R₁ et R'₁ ont la même signification que précédemment,
qui est hydrolysée en présence d'un hydroxyde de métal alcalin en milieu aqueux ou hydroalcoolique,
pour conduire à l'acide de formule (VIII) : 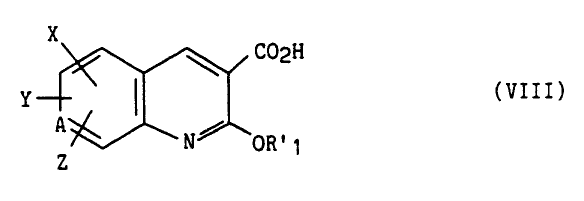 dans laquelle X, Y, Z, A et R'₁ ont la même signification que précédemment,
qui est :
a transformé en azoture d'acide correspondant :
- par activation de la fonction acide sous forme d'anhydride ou d'halogènure d'acide puis réaction avec de l'azoture de sodium dans un solvant inerte,
- ou par réaction, dans un solvant inerte, avec de l'azoture de diphénoxyphosphoryl en présence d'une amine tertiaire,
b réarrangé, par chauffage, dans un solvant inerte, en milieu anhydre en additionnant à la fin du dégagement gazeux un excès d'un alcool de formule R''₁OH (dans laquelle R''₁ a la même signification que R₁) ou par chauffage direct dans l'alcool R''₁OH,
pour conduire au carbamate correspondant de formule (IX) : 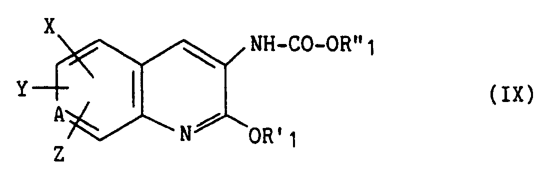 dans laquelle X, Y, Z, A, R'₁, R''₁ ont la même signification que précédemment,
qui est soit hydrolysé en milieu acide anhydre, soit hydrogéné en milieu anhydre par de l'hydrogène en présence d'un catalyseur,
pour conduire à l'amine de formule (X) : 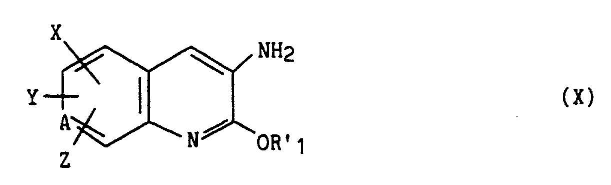 dans laquelle X, Y, Z, A et R'₁ ont la même signification que précédemment,
qui subit une sulfonylation par réaction avec un halogénure ou un anhydride de sulfonyle de formules respectives RSO₂W ou (RSO₂)₂O (dans lesquelles R a la même signification que dans la formule (I) et W représente un atome d'halogène), en présence d'une amine tertiaire, en milieu aprotique,
pour conduire à la sulfonamide de formule (XI) : 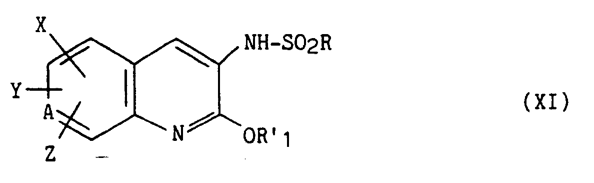 dans laquelle X, Y, Z, A, R et R'₁ ont la même signification que précédemment,
qui est déprotégée, selon la nature du groupement R'₁, par chauffage en milieu aqueux acide ou par hydrogénation catalytique,
pour conduire au composé de formule (I),
2°/ 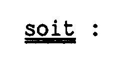 avec un nitroacétate d'alkyle en milieu protique, en présence d'un alcoolate de métal alcalin, d'une amine tertiaire ou d'un hydroxyde de métal alcalin, ou en milieu inerte à haut point d'ébullition en présence d'une amine tertiaire ou secondaire au reflux du solvant,
pour conduire à la 3-nitro-2-(1H)-quinoléinone de formule (XII) : 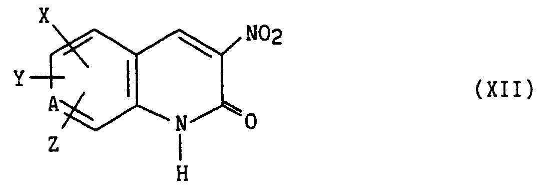 dans laquelle X, Y, Z et A ont la même signification que dans la formule (I),
qui est protégée, selon les mêmes techniques que celles décrites pour la protection de la quinoléinone de formule (VI) en 2-alkoxyquinoléine de formule (VII),
pour conduire à la 2-alkoxyquinoléine de formule (XIII) : 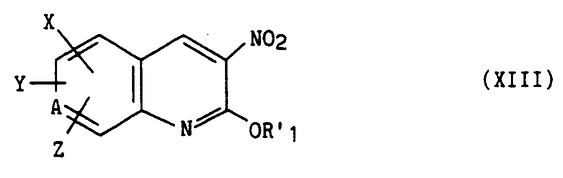 dans laquelle X, Y, Z, A et R'₁ ont la même signification que dans la formule (I),
dont le groupe nitro est réduit en présence de chlorure d'étain dans un alcool, d'hydrosulfite de sodium dans l'eau ou par hydrogénation catalytique,
pour conduire à l'amine de formule (X) définie précédemment,
qui subit la sulfonylation puis la déprotection décrites plus haut permettant d'obtenir un composé de formule (I),
3°/ 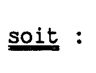 avec un dérivé de la glycine protégée de formule (XIV), activé sous forme d'anhydride, d'azoture, de cyanure ou d'halogénure correspondant, en milieu inerte, en présence de triéthylamine : 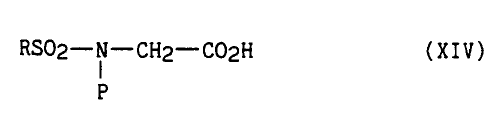 dans laquelle R a la même signification que dans la formule (I) et P représente un groupement protecteur classique de la fonction amine d'une amino-acide,
pour conduire à l'aldéhyde de formule (XV) : 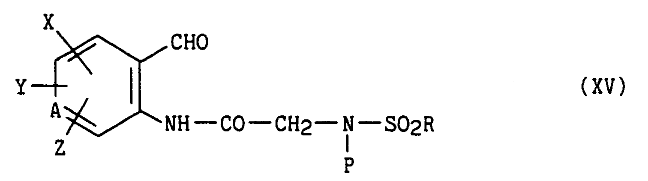 dans laquelle X, Y, Z, A, P et R ont la même signification que précédemment,
qui est cyclisé, en milieu alcoolique, en présence d'un alcoolate de métal alcalin, d'une amine tertiaire ou d'un hydroxyde de métal alcalin,
pour conduire à la quinoléinone de formule (XVI) : 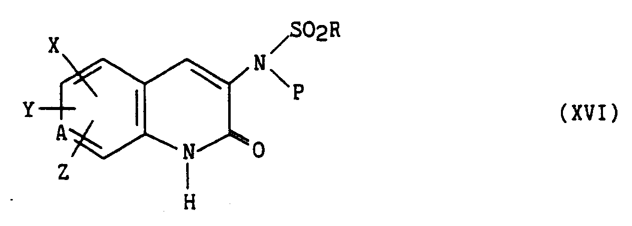 dans laquelle X, Y, Z, A, R et P ont la même signification que précédemment,
qui est déprotégé en présence de chlorure d'aluminium dans un solvant inerte ou par hydrogénation catalytique,
pour-conduire à un composé de formule (I),
composé de formule (I),
- qui, lorsque X et/ou Y et/ou Z représentent un atome d'hydrogène, peut subir des substitutions électrophiles, selon des techniques classiques de substitution des noyaux aromatiques, conduisant à un composé de formule (I) mono, di ou trisubstitué sur le noyau phényl de la quinoléinone,
- qui, lorsque X, Y ou Z représentent un groupement nitro, peut être transformé en dérivé amino ou acylamino correspondant, dérivé amino qui lui-même peut être transformé en dérivé azido correspondant selon des techniques classiques de la chimie organique,
- qui, lorsque X représente un groupement nitro et Y, situé sur un carbone adjacent représente un groupement azido, peuvent subir une cyclisation pour conduire au dérivé 1,2,5-oxadiazolo correspondant,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutiquement acceptable.

8. Compositions pharmaceutiques contenant comme principe actif au moins un composé selon l'une quelconque des revendications 1 à 6, seul ou en combinaison avec un ou plusieurs véhicules inertes, non toxiques, pharmaceutiquement acceptables.

9. Compositions pharmaceutiques selon la revendication 8 contenant au moins un principe actif selon l'une des revendications 1 à 6 utiles en tant qu'inhibiteurs des phénomènes pathologiques liés à l'hyperactivation des voies de neurotransmission aux aminoacides excitateurs.

## Revendications (Revendications pour l'(les) Etat(s) contractant(s) suivant(s): ES)

1. Procédé de préparation des composés de formule (I) : 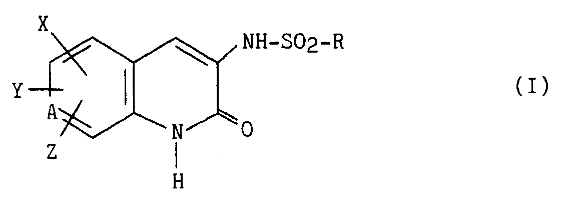 dans laquelle :
X, Y, Z :
- identiques ou différents, représentent un atome d'hydrogène, un atome d'halogène, un groupement nitro, cyano, azido, trihalogénométhyle, alkyle (C₁-C₆) linéaire ou ramifié, alkoxy (C₁-C₆) linéaire ou ramifié, amino (substitué ou non par un groupement acyl (C₁-C₆) linéaire ou ramifié),
- ou bien, lorsqu' ils sont situés sur deux carbones adjacents, forment avec les atomes de carbone auxquels ils sont attachés, un cycle phényle ou un hétérocycle à 5 ou 6 chaînons comportant de 1 à 3 hétéroatomes,
R représente un groupement alkyle (C₁-C₆) linéaire ou ramifié, trihalogénométhyle, phényle (substitué ou non par un ou plusieurs atomes d'halogène ou groupements alkyle (C₁-C₆) linéaire ou ramifié, trihalogénométhyle, alkoxy (C₁-C₆) linéaire ou ramifié), 2-thiényle (substitué ou non par un atome d'halogène), naphtyle ou styryle,
A représente un radical CH ou un atome d'azote,
leurs énantiomères, diastéréoisomères et épimères ainsi que leurs sels d'addition à une base pharmaceutiquement acceptable,
caractérisé en ce que l'on utilise comme produit de départ un composé de formule (II) : 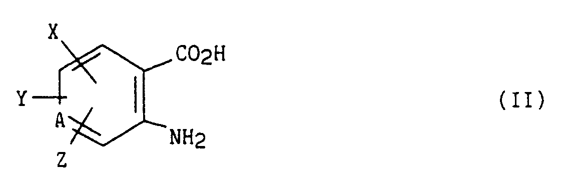 dans laquelle X, Y, Z et A ont la même signification que dans la formule (I),
que l'on réduit selon la nature des substituants X, Y et Z ,
soit en milieu aprotique en présence d'hydrure de lithium aluminium, d'hydrure d'aluminium, de diborane ou des complexes de borane,
soit en milieu protique acide lorsque l'on utilise le cyanoborohydrure de sodium,
pour conduire à un alcool de formule (III) : 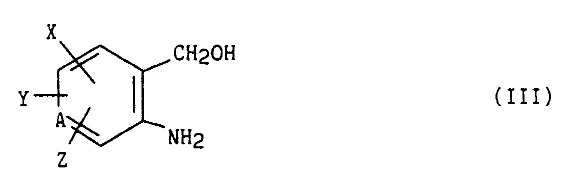 dans laquelle X, Y, Z et A ont la même signification que dans la formule (I)
que l'on oxyde à l'aide d'un oxyde métallique dans un solvant inerte, d'un halogénate de métal alcalin dans un solvant protique ou d'un chlorure d'acide dans le diméthylsulfoxyde,
pour conduire à l'adéhyde de formule (IV) : 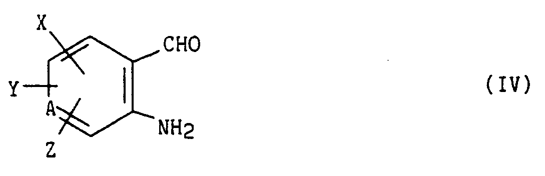 dans laquelle X, Y, Z et A ont la même signification que dans la formule (I),
qui est alors condensé :
1°/ 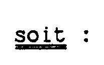 avec un malonate d'alkyle de formule (V), en milieu protique, en présence d'un alcoolate de métal alcalin, d'une amine tertiaire ou d'un hydroxyde de métal alcalin : 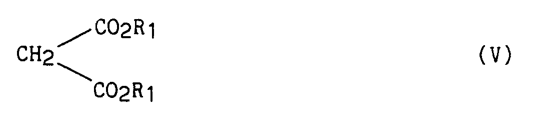 dans laquelle R₁ représente un groupement alkyle (C₁-C₆) linéaire ou ramifié, substitué ou non par un ou plusieurs groupements phényle,
pour conduire à une 2-(1H)-quinoléinone de formule (VI) : 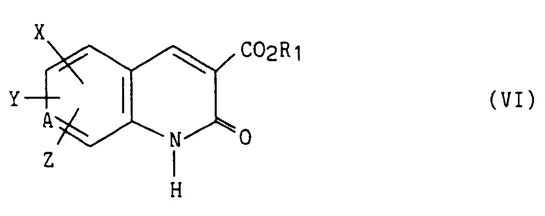 dans laquelle X, Y, Z, A et R₁ ont la même signification que précédemment, qui est protégée :
- par réaction avec un tétrafluoroborate de trialkyloxonium dans de l'éther ou le dichlorométhane,
- ou par réaction, dans un solvant inerte ou sans, d'un oxyhalogénure de phosphore, d'un pentahalogénure de phosphore, d'un halogénure de thionyle, de phosgène, de thiophosgène, de phosgène imminium ou de trimère de phosgène, suivie d'une réaction de la 2-halogénoquinoléine ainsi formée avec un alkoxyde de métal alcalin de formule R'₁OM (dans laquelle R'₁ a la même signification que R₁ et M représente un métal alcalin), en milieu alcoolique,
- ou par réaction avec l'hydrure de sodium, le butyllithium, un alcoolate de métal alcalin en présence d'un agent d'alkylation de formule R'₁V (dans laquelle R'₁ a la même signification que précédemment et V est un groupe partant),
pour conduire à la 2-alkoxyquinoléine de formule (VII) : 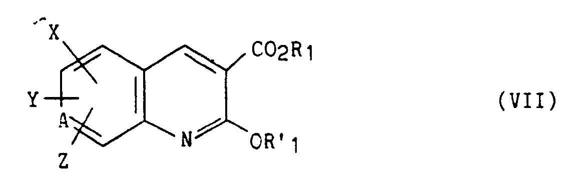 dans laquelle X, Y, Z, A, R₁ et R'₁ ont la même signification que précédemment,
qui est hydrolysée en présence d'un hydroxyde de métal alcalin en milieu aqueux ou hydroalcoolique,
pour conduire à l'acide de formule (VIII) : 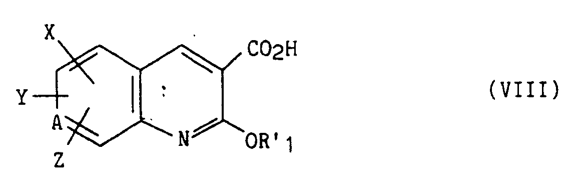 dans laquelle X, Y, Z, A et R'₁ ont la même signification que précédemment,
qui est :
a transformé en azoture d'acide correspondant :
- par activation de la fonction acide sous forme d'anhydride ou d'halogènure d'acide puis réaction avec de l'azoture de sodium dans un solvant inerte,
- ou par réaction, dans un solvant inerte, avec de l'azoture de diphénoxyphosphoryl en présence d'une amine tertiaire,
b réarrangé, par chauffage, dans un solvant inerte, en milieu anhydre en additionnant à la fin du dégagement gazeux un excès d'un alcool de formule R''₁OH (dans laquelle R''₁ a la même signification que R₁) ou par chauffage direct dans l'alcool R''₁OH,
pour conduire au carbamate correspondant de formule (IX) : 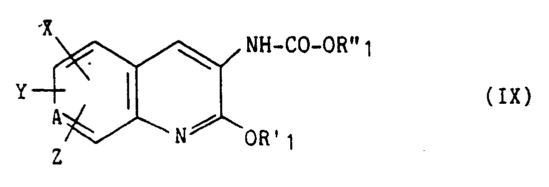 dans laquelle X, Y, Z, A, R'₁, R''₁ ont la même signification que précédemment,
qui est soit hydrolysé en milieu acide anhydre, soit hydrogéné en milieu anhydre par de l'hydrogène en présence d'un catalyseur,
pour conduire à l'amine de formule (X) : 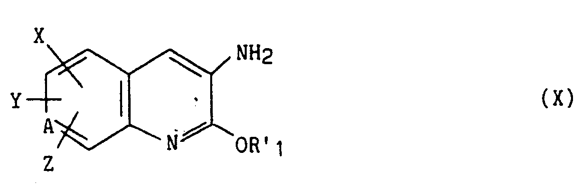 dans laquelle X, Y, Z, A et R'₁ ont la même signification que précédemment,
qui subit une sulfonylation par réaction avec un halogénure ou un anhydride de sulfonyle de formules respectives RSO₂W ou (RSO₂)₂O (dans lesquelles R a la même signification que dans la formule (I) et W représente un atome d'halogène), en présence d'une amine tertiaire, en milieu aprotique,
pour conduire à la sulfonamide de formule (XI) : 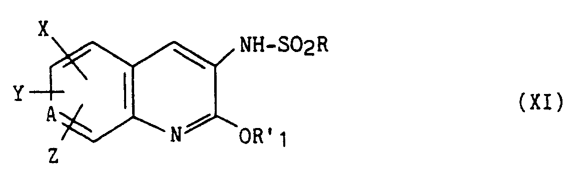 dans laquelle X, Y, Z, A, R et R'₁ ont la même signification que précédemment,
qui est déprotégée, selon la nature du groupement R'₁, par chauffage en milieu aqueux acide ou par hydrogénation catalytique,
pour conduire au composé de formule (I),
2°/ 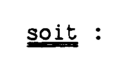 avec un nitroacétate d'alkyle en milieu protique, en présence d'un alcoolate de métal alcalin, d'une amine tertiaire ou d'un hydroxyde de métal alcalin, ou en milieu inerte à haut point d'ébullition en présence d'une amine tertiaire ou secondaire au reflux du solvant,
pour conduire à la 3-nitro-2(1H)quinoléinone de formule (XII) : 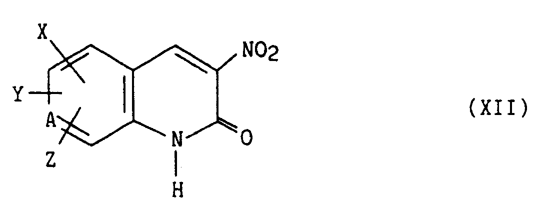 dans laquelle X, Y, Z et A ont la même signification que dans la formule (I),
qui est protégée, selon les mêmes techniques que celles décrites pour la protection de la quinoléinone de formule (VI) en 2-alkoxyquinoléine de formule (VII),
pour conduire à la 2-alkoxyquinoléine de formule (XIII) : 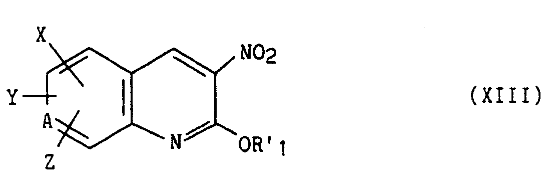 dans laquelle X, Y, Z, A et R'₁ ont la même signification que dans la formule (I),
dont le groupe nitro est réduit en présence de chlorure d'étain dans un alcool, d'hydrosulfite de sodium dans l'eau ou par hydrogénation catalytique,
pour conduire à l'amine de formule (X) définie précédemment,
qui subit la sulfonylation puis la déprotection décrites plus haut permettant d'obtenir un composé de formule (I),
3°/ 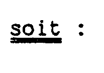 avec un dérivé de la glycine protégée de formule (XIV), activé sous forme d'anhydrlde, d'azoture, de cyanure ou d'halogénure correspondant, en milieu inerte, en présence de triéthylamine : 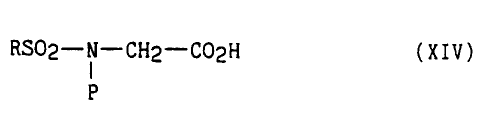
dans laquelle R a la même signification que-dans la formule (I) et P représente un groupement protecteur classique de la fonction amine d'une amino-acide,
pour conduire à l'aldéhyde de formule (XV) : 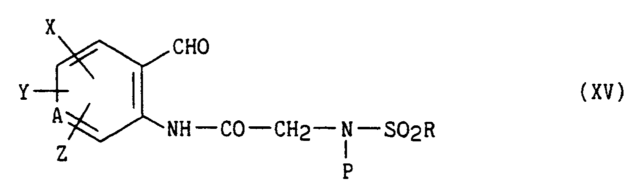 dans laquelle X, Y, Z, A, P et R ont la même signification que précédemment,
qui est cyclisé, en milieu alcoolique, en présence d'un alcoolate de métal alcalin, d'une amine tertiaire ou d'un hydroxyde de métal alcalin,
pour conduire à la quinoléinone de formule (XVI) : 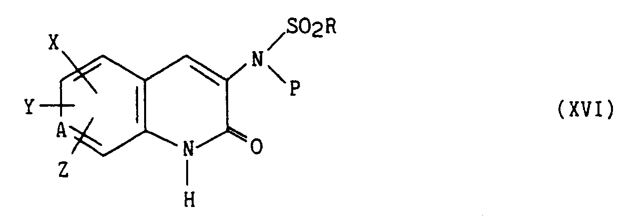 dans laquelle X, Y, Z, A, R et P ont la même signification que précédemment,
qui est déprotégé en présence de chlorure d'aluminium dans un solvant inerte ou par hydrogénation catalytique,
pour conduire à un composé de formule (I),
composé de formule (I),
- qui, lorsque X et/ou Y et/ou Z représentent un atome d'hydrogène, peut subir des substitutions électrophiles, selon des techniques classiques de substitution des noyaux aromatiques, conduisant à un composé de formule (I) mono, di ou trisubstitué sur le noyau phényl de la quinoléinone,
- qui, lorsque X, Y ou Z représentent un groupement nitro, peut être transformé en dérivé amino ou acylamino correspondant, dérivé amino qui lui-même peut être transformé en dérivé azido correspondant selon des techniques classiques de la chimie organique,
- qui, lorsque X représente un groupement nitro et Y, situé sur un carbone adjacent représente un groupement azido, peuvent subir une cyclisation pour conduire au dérivé 1,2,5-oxadiazolo correspondant,
- qui peut être, le cas échéant, purifié selon une technique classique de purification,
- dont on sépare, le cas échéant, les isomères selon une technique classique de séparation,
- que l'on transforme, si on le souhaite, en ses sels d'addition à une base pharmaceutlquement acceptable.

2. Procédé de préparation des composés de formule (I) selon la revendication 1 tels que R représente un groupement trihalogénométhyle.

3. Procédé de préparation des composés de formule (I) selon la revendication 1 tels que l'un au moins des groupements X, Y ou Z représente un groupement nitro.

4. Procédé de préparation des composés de formule (I) selon la revendication 1 tels que l'un au moins des groupements X, Y ou Z représente un groupement chloro.

5. Procédé de préparation des composés de formule (I) selon la revendication 1 tels que X, Y ou Z lorsqu'ils sont situés sur deux carbones adjacents forment avec les atomes de carbone auxquels ils sont attachés un cycle 1,2,5-oxadiazole.

6. Procédé de préparation du composé de formule (I) selon la revendication 1 qui est le 7-nitro-3(trifluorométhylsulfonylamino)-2-(1H)-quinoléinone.

## Claims (Claims for the following Contracting State(s): AT, BE, CH, LI, DE, DK, FR, GB, GR, IE, IT, LU, NL, PT, SE)

1. Compounds of formula (I): 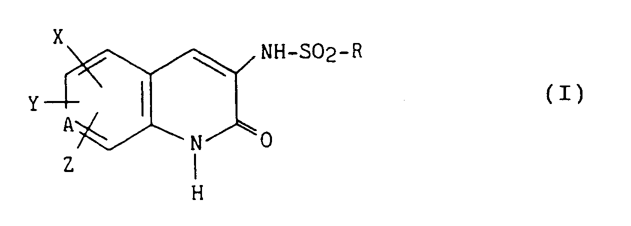 wherein:
X, Y, Z:
- which may be identical or different, represent a hydrogen atom, a halogen atom, a nitro grouping, a cyano grouping, an azido grouping, a trihalomethyl grouping, a linear or branched (C₁-C₆)-alkyl grouping, a linear or branched (C₁-C₆)-alkoxy grouping or an amino grouping (unsubstituted or substituted by a linear or branched (C₁-C₆)-acyl grouping),
- or, when they are positioned on two adjacent carbon atoms, they form with the carbon atoms to which they are attached a phenyl ring or a heterocycle having 5 or 6 ring members and containing from 1 to 3 hetero atoms,
R represents a linear or branched (C₁-C₆)-alkyl grouping, a trihalomethyl grouping, a phenyl grouping (unsubstituted or substituted by one or more halogen atoms or linear or branched (C₁-C₆)-alkyl groupings, trihalomethyl groupings or linear or branched (C₁-C₆)-alkoxy groupings), a 2-thienyl grouping (unsubstituted or substituted by a halogen atom), a naphthyl grouping or a styryl grouping, and
A represents a CH radical or a nitrogen atom,
their enantiomers, diastereoisomers and epimers and also their addition salts with a pharmaceutically acceptable base.

2. Compounds of formula (I) according to claim 1 such that R represents a trihalomethyl grouping.

3. Compounds of formula (I) according to claim 1 such that at least one of the groupings X, Y and Z represents a nitro grouping.

4. Compounds of formula (I) according to claim 1 such that at least one of the groupings X, Y and Z represents a chloro grouping.

5. Compounds of formula (I) according to claim 1 such that X, Y or Z, when they are positioned on two adjacent carbon atoms, form with the carbon atoms to which they are attached a 1,2,5-oxadiazole ring.

6. Compound of formula (I) according to claim 1 which is 7-nitro-3-(trifluoromethylsulphonylamino)-2-(1H)-quinolinone.

7. Process for the preparation of the compounds of formula (I), characterised in that there is used as starting material a compound of formula (II): 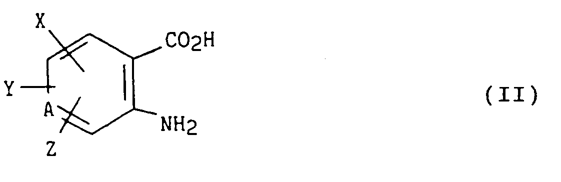 wherein X, Y, Z and A are as defined in formula (I),
which is reduced, depending on the nature of the substituents X, Y and Z,
either in an aprotic medium in the presence of lithium aluminium hydride, aluminium hydride, diborane or borane complexes,
or in a protic acid medium when sodium cyanoborohydride is used,
to yield an alcohol of formula (III): 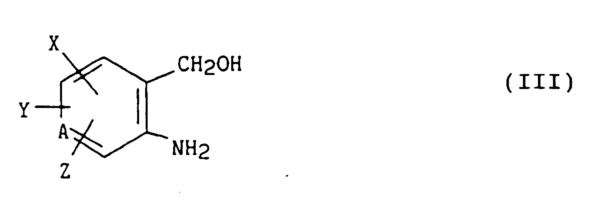 wherein X, Y, Z and A are as defined in formula (I),
which is oxidised by means of a metal oxide in an inert solvent, by means of an alkali metal halogenate in a protic solvent or by means of an acid chloride in dimethyl sulphoxide,
to yield the aldehyde of formula (IV): 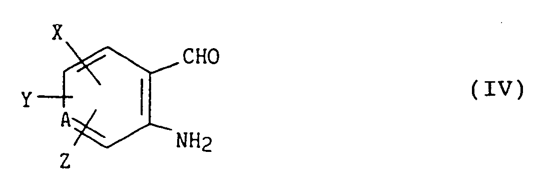 wherein X, Y, Z and A are as defined in formula (I),
which is then condensed:
1^{o}/ either:
with an alkyl malonate of formula (V), in a protic medium, in the presence of an alkali metal alcoholate, a tertiary amine or an alkali metal hydroxide: 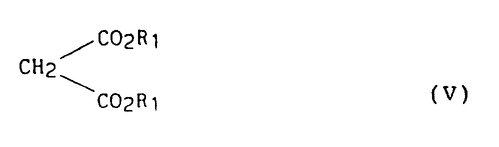 wherein R₁ represents a linear or branched (C₁-C₆)-alkyl grouping that is unsubstituted or substituted by one or more phenyl groupings,
to yield a 2-(1H)-quinolinone of formula (VI): 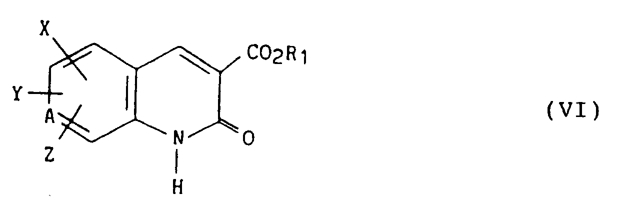 wherein X, Y, Z, A and R₁ are as defined above, which is protected:
- by reaction with a trialkyloxonium tetrafluoroborate in ether or dichloromethane,
- or by reaction, in an inert solvent or without a solvent, with a phosphorus oxyhalide, a phosphorus pentahalide, a thionyl halide, phosgene, thiophosgene, a phosgene iminium halide or trimeric phosgene, followed by reaction of the resulting 2-haloquinoline with an alkali metal alkoxide of the formula R'₁OM (wherein R'₁ is as defined for R₁ and M represents an alkali metal), in an alcoholic medium,
- or by reaction with sodium hydride, butyl lithium, or an alkali metal alcoholate in the presence of an alkylating agent of the formula R'₁V (wherein R'₁ is as defined above and V is a leaving group),
to yield the 2-alkoxyquinoline of formula (VII): 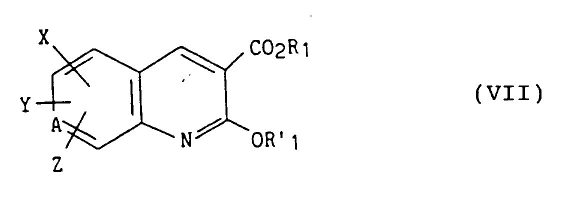 wherein X, Y, Z, A, R₁ and R'₁ are as defined above,
which is hydrolysed in the presence of an alkali metal hydroxide in an aqueous or aqueous alcoholic medium,
to yield the acid of formula (VIII): 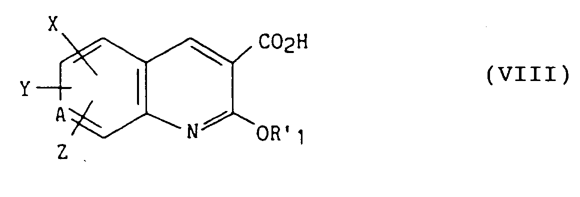 wherein X, Y, Z, A and R'₁ are as defined above,
which is:
a converted into the corresponding acid azide:
- by activation of the acid function to the acid anhydride or acid halide form and then reaction with sodium azide in an inert solvent,
- or by reaction, in an inert solvent, with diphenoxyphosphoryl azide in the presence of a tertiary amine,
b rearranged, by heating, in an inert solvent, in an anhydrous medium, by adding, when the evolution of gas is complete, an excess of an alcohol of the formula R''₁OH (wherein R''₁ is as defined for R₁) or by heating directly in the alcohol R''₁OH,
to yield the corresponding carbamate of formula (IX): 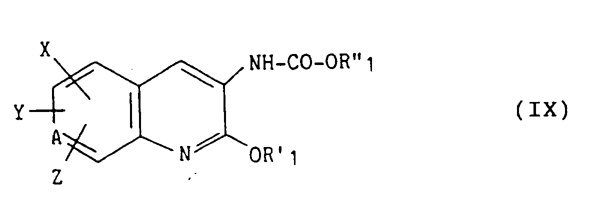 wherein X, Y, Z, A, R'₁ and R''₁ are as defined above,
which is either hydrolysed in an anhydrous acid medium, or hydrogenated in an anhydrous medium by hydrogen in the presence of a catalyst,
to yield the amine of formula (X): 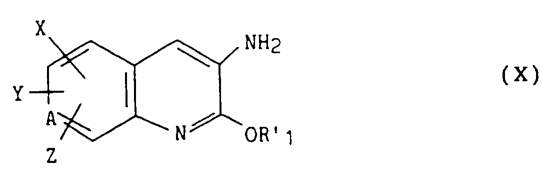 wherein X, Y, Z, A and R'₁ are as defined above,
which undergoes sulphonylation by reaction with a sulphonyl halide or anhydride of the respective formulae RSO₂W and (RSO₂)₂O (wherein R is as defined in formula (I) and W represents a halogen atom), in the presence of a tertiary amine, in an aprotic medium,
to yield the sulphonamide of formula (XI): 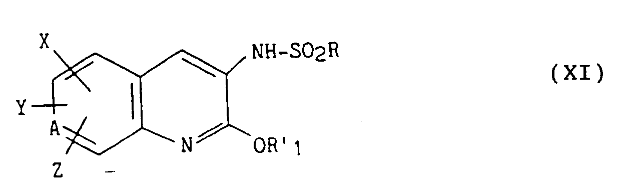 wherein X, Y, Z, A, R and R'₁ are as defined above,
which is deprotected, depending on the nature of the grouping R'₁, by heating in an acidic aqueous medium or by catalytic hydrogenation,
to yield the compound of formula (I),
2^{o}/ or:
with an alkyl nitroacetate in a protic medium, in the presence of an alkali metal alcoholate, a tertiary amine or an alkali metal hydroxide, or in an inert medium having a high boiling point in the presence of a tertiary or secondary amine at the reflux temperature of the solvent,
to yield the 3-nitro-2-(1H)-quinolinone of formula (XII): 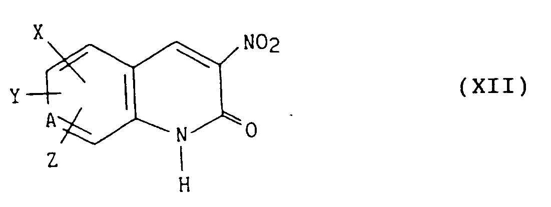 wherein X, Y, Z and A are as defined in formula (I),
which is protected in accordance with the same techniques as those described for the protection of the quinolinone of formula (VI) to form the 2-alkoxyquinoline of formula (VII),
to yield the 2-alkoxyquinoline. of formula (XIII): 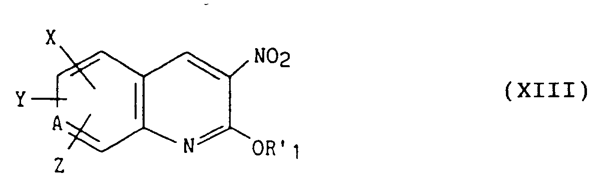 wherein X, Y, Z, A and R'₁ are as defined in formula (I),
the nitro group of which is reduced in the presence of tin chloride in an alcohol or sodium hydrosulphite in water or by catalytic hydrogenation to yield the amine of formula (X) defined above,
which undergoes the sulphonylation and then the deprotection described above enabling a compound of formula (I) to be obtained,
3^{o}/ or:
with a protected glycine compound of formula (XIV), activated in the form of the corresponding anhydride, azide, cyanide or halide, in an inert medium, in the presence of triethylamine: 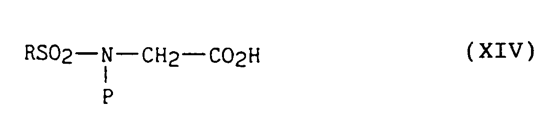 wherein R is as defined in formula (I) and P represents a customary protecting grouping for the amine function of an amino acid,
to yield the aldehyde of formula (XV): 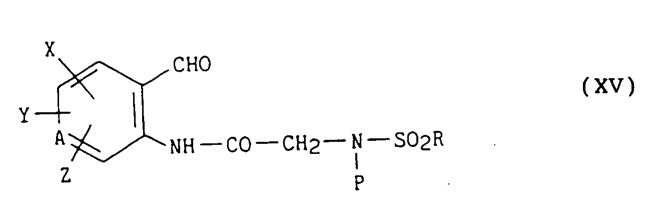 wherein X, Y, Z, A, P and R are as defined above,
which is cyclised, in an alcoholic medium, in the presence of an alkali metal alcoholate, a tertiary amine or an alkali metal hydroxide,
to yield the quinolinone of formula (XVI): 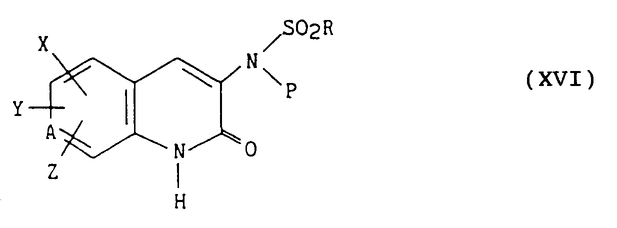 wherein X, Y, Z, A, R and P are as defined above,
which is deprotected in the presence of aluminium chloride in an inert solvent or by catalytic hydrogenation,
to yield a compound of formula (I),
compound of formula (I),
- which, when X and/or Y and/or Z represent a hydrogen atom, may undergo electrophilic substitutions, in accordance with customary techniques of substituting aromatic nuclei, leading to a compound of formula (I) mono-, di- or tri-substituted on the phenyl ring of the quinolinone,
- which, when X, Y or Z represents a nitro grouping, may be converted into the corresponding amino or acylamino compound, which amino compound may itself be converted into the corresponding azido compound in accordance with customary techniques of organic chemistry,
- which, when X represents a nitro grouping and Y, positioned on an adjacent carbon atom, represents an azido grouping, may undergo cyclisation to yield the corresponding 1,2,5-oxadiazole compound,
- which, where appropriate, may be purified in accordance with a customary purification technique,
- of which, where appropriate, the isomers are separated in accordance with a customary separation technique,
- which is converted, if desired, into its addition salts with a pharmaceutically acceptable base.

8. Pharmaceutical compositions containing as active ingredient at least one compound according to any one of claims 1 to 6, alone or in combination with one or more inert, non-toxic pharmaceutically acceptable carriers.

9. Pharmaceutical compositions according to claim 8, containing at least one active ingredient according to one of claims 1 to 6 for use as inhibitors of pathological phenomena associated with the hyperactivation of the neurotransmission pathways by the exciting amino acids.

## Claims (Claims for the following Contracting State(s): ES)

1. Process for the preparation of the compounds of formula (I):
wherein: 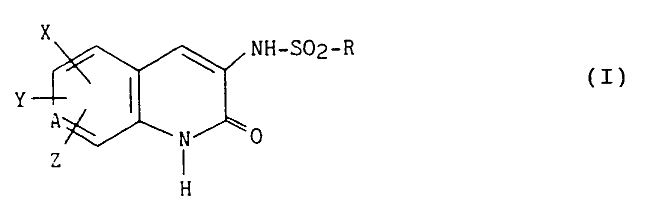
X, Y, Z:
- which may be identical or different, represent a hydrogen atom, a halogen atom, a nitro grouping, a cyano grouping, an azido grouping, a trihalomethyl grouping, a linear or branched (C₁-C₆)-alkyl grouping, a linear or branched (C₁-C₆)-alkoxy grouping or an amino grouping (unsubstituted or substituted by a linear or branched (C₁-C₆)-acyl grouping),
- or, when they are positioned on two adjacent carbon atoms, they form with the carbon atoms to which they are attached a phenyl ring or a heterocycle having 5 or 6 ring members and containing from 1 to 3 hetero atoms,
R represents a linear or branched (C₁-C₆)-alkyl grouping, a trihalomethyl grouping, a phenyl grouping (unsubstituted or substituted by one or more halogen atoms or linear or branched (C₁-C₆)-alkyl groupings, trihalomethyl groupings or linear or branched (C₁-C₆)-alkoxy groupings), a 2-thienyl grouping (unsubstituted or substituted by a halogen atom), a naphthyl grouping or a styryl grouping, and
A represents a CH radical or a nitrogen atom,
their enantiomers, diastereoisomers and epimers and also their addition salts with a pharmaceutically acceptable base,
characterised in that there is used as starting material a compound of formula (II): 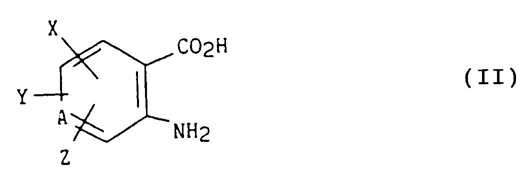 wherein X, Y, Z and A are as defined in formula (I),
which is reduced, depending on the nature of the substituents X, Y and Z,
either in an aprotic medium in the presence of lithium aluminium hydride, aluminium hydride, diborane or borane complexes,
or in a protic acid medium when sodium cyanoborohydride is used,
to yield an alcohol of formula (III): 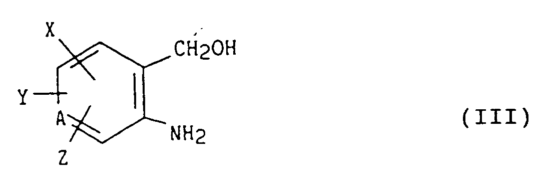 wherein X, Y, Z and A are as defined in formula (I),
which is oxidised by means of a metal oxide in an inert solvent, by means of an alkali metal halogenate in a protic solvent or by means of an acid chloride in dimethyl sulphoxide,
to yield the aldehyde of formula (IV): 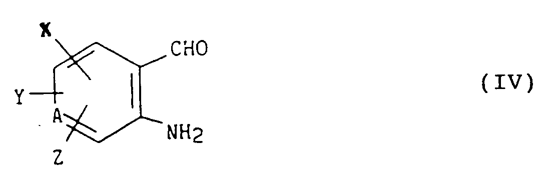 wherein X, Y, Z and A are as defined in formula (I),
which is then condensed:
1^{o}/ either:
with an alkyl malonate of formula (V), in a protic medium, in the presence of an alkali metal alcoholate, a tertiary amine or an alkali metal hydroxide: 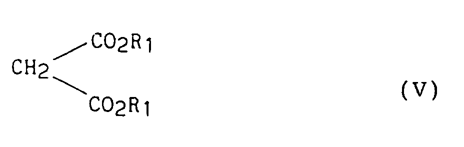 wherein R₁ represents a linear or branched (C₁-C₆)-alkyl grouping that is unsubstituted or substituted by one or more phenyl groupings,
to yield a 2-(1H)-quinolinone of formula (VI): 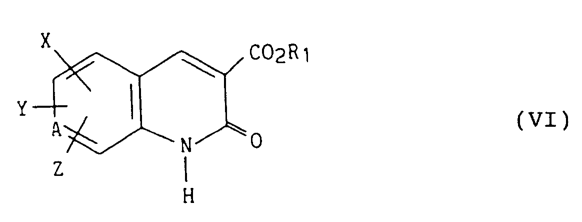 wherein X, Y, Z, A and R₁ are as defined above,
which is protected:
- by reaction with a trialkyloxonium tetrafluoroborate in ether or dichloromethane,
- or by reaction, in an inert solvent or without a solvent, with a phosphorus oxyhalide, a phosphorus pentahalide, a thionyl halide, phosgene, thiophosgene, a phosgene iminium halide or trimeric, halide, followed by reaction of the resulting 2-haloquinoline with an alkali metal alkoxide of the formula R'₁OM (wherein R'₁ is as defined for R₁ and M represents an alkali metal), in an alcoholic medium,
- or by reaction with sodium hydride, butyl lithium, or an alkali metal alcoholate in the presence of an alkylating agent of the formula R'₁V (wherein R'₁ is as defined above and V is a leaving group),
to yield the 2-alkoxyquinoline of formula (VII): 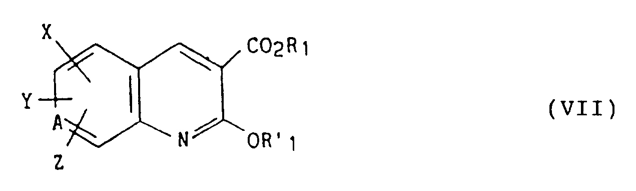 wherein X, Y, Z, A, R₁ and R'₁ are as defined above,
which is hydrolysed in the presence of an alkali metal hydroxide in an aqueous or aqueous alcoholic medium,
to yield the acid of formula (VIII): 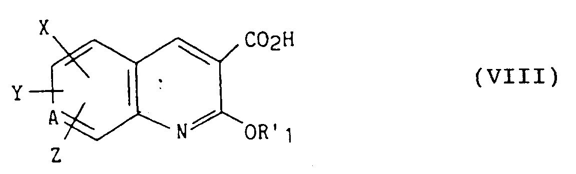 wherein X, Y, Z, A and R'₁ are as defined above,
which is:
a converted into the corresponding acid azide:
- by activation of the acid function to the acid anhydride or acid halide form and then reaction with sodium azide in an inert solvent,
- or by reaction, in an inert solvent, with diphenoxyphosphoryl azide in the presence of a tertiary amine,
b rearranged, by heating, in an inert solvent, in an anhydrous medium, by adding, when the evolution of gas is complete, an excess of an alcohol of the formula R''₁OH (wherein R''₁ is as defined for R₁) or by heating directly in the alcohol R''₁OH,
to yield the corresponding carbamate of formula (IX): 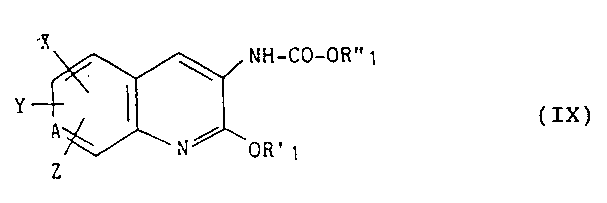 wherein X, Y, Z, A, R'₁ and R''₁ are as defined above,
which is either hydrolysed in an anhydrous acid medium, or hydrogenated in an anhydrous medium by hydrogen in the presence of a catalyst,
to yield the amine of formula (X): 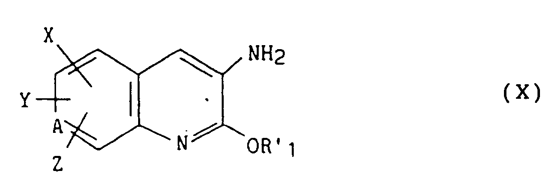 wherein X, Y, Z, A and R'₁ are as defined above,
which undergoes sulphonylation by reaction with a sulphonyl halide or anhydride of the respective formulae RSO₂W and (RSO₂)₂O (wherein R is as defined in formula (I) and W represents a halogen atom), in the presence of a tertiary amine, in an aprotic medium,
to yield the sulphonamide of formula (XI): 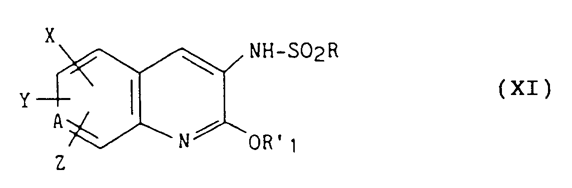 wherein X, Y, Z, A, R and R'₁ are as defined above,
which is deprotected, depending on the nature of the grouping R'₁, by heating in an acidic aqueous medium or by catalytic hydrogenation,
to yield the compound of formula (I),
2^{o}/ or:
with an alkyl nitroacetate in a protic medium, in the presence of an alkali metal alcoholate, a tertiary amine or an alkali metal hydroxide, or in an inert medium having a high boiling point in the presence of a tertiary or secondary amine at the reflux temperature of the solvent,
to yield the 3-nitro-2-(1H)-quinolinone of formula (XII): 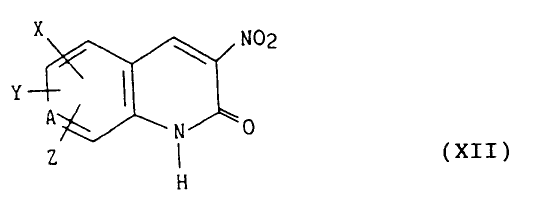 wherein X, Y, Z and A are as defined in formula (I),
which is protected in accordance with the same techniques as those described for the protection of the quinolinone of formula (VI) to form the 2-alkoxyquinoline of formula (VII),
to yield the 2-alkoxyquinoline of formula (XIII): 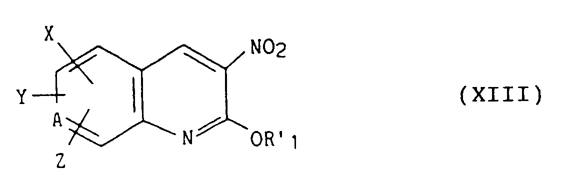 wherein X, Y, Z, A and R'₁ are as defined in formula (I),
the nitro group of which is reduced in the presence of tin chloride in an alcohol or sodium hydrosulphite in water or by catalytic hydrogenation to yield the amine of formula (X) defined above,
which undergoes the sulphonylation and then the deprotection described above enabling a compound of formula (I) to be obtained,
3^{o}/ or:
with a protected glycine compound of formula (XIV), activated in the form of the corresponding anhydride, azide, cyanide or halide, in an inert medium, in the presence of triethylamine: 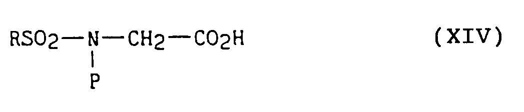 wherein R is as defined in formula (I) and P represents a customary protecting grouping for the amine function of an amino acid,
to yield the aldehyde of formula (XV): 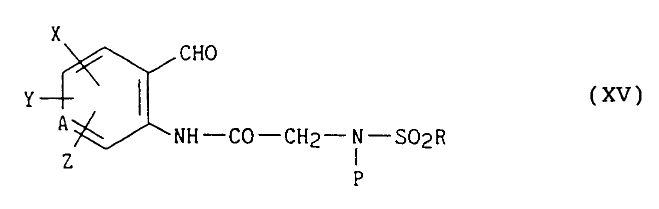 wherein X, Y, Z, A, P and R are as defined above,
which is cyclised, in an alcoholic medium, in the presence of an alkali metal alcoholate, a tertiary amine or an alkali metal hydroxide,
to yield the quinolinone of formula (XVI): 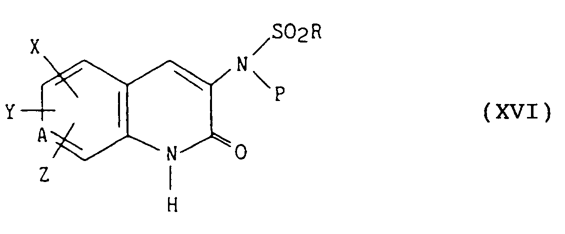 wherein X, Y, Z, A, R and P are as defined above,
which is deprotected in the presence of aluminium chloride in an inert solvent or by catalytic hydrogenation,
to yield a compound of formula (I),
compound of formula (I),
- which, when X and/or Y and/or Z represent a hydrogen atom, may undergo electrophilic substitutions, in accordance with customary techniques of substituting aromatic nuclei, leading to a compound of formula (I) mono-, di- or tri-substituted on the phenyl ring of the quinolinone,
- which, when X, Y or Z represents a nitro grouping, may be converted into the corresponding amino or acylamino compound, which amino compound may itself be converted into the corresponding azido compound in accordance with customary techniques of organic chemistry,
- which, when X represents a nitro grouping and Y, positioned on an adjacent carbon atom, represents an azido grouping, may undergo cyclisation to yield the corresponding 1,2,5-oxadiazole compound,
- which, where appropriate, may be purified in accordance with a customary purification technique,
- of which, where appropriate, the isomers are separated in accordance with a customary separation technique,
- which is converted, if desired, into its addition salts with a pharmaceutically acceptable base.

2. Process for the preparation of the compounds of formula (I) according to claim 1 such that R represents a trihalomethyl grouping.

3. Process for the preparation of the compounds of formula (I) according to claim 1 such that at least one of the groupings X, Y and Z represents a nitro grouping.

4. Process for the preparation of the compounds of formula (I) according to claim 1 such that at least one of the groupings X, Y and Z represents a chloro grouping.

5. Process for the preparation of the compounds of formula (I) according to claim 1 such that X, Y or Z, when they are positioned on two adjacent carbon atoms, form with the carbon atoms to which they are attached a 1,2,5-oxadiazole ring.

6. Process for the preparation of the compound of formula (I) according to claim 1 which is 7-nitro-3-(trifluoromethylsulphonylamino)-2-(1H)-quinolinone.

## Patentansprüche (Patentansprüche für folgende(n) Vertragsstaat(en): AT, BE, CH, LI, DE, DK, FR, GB, GR, IE, IT, LU, NL, PT, SE)

1. Verbindungen der Formel (I): 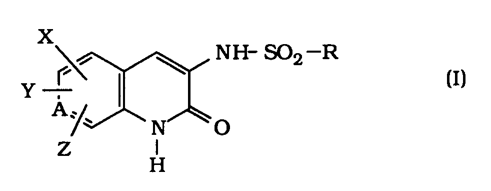 in der:
X, Y, Z:
- die gleichartig oder verschieden sein können, ein Wasserstoffatom, ein Halogenatom, eine Nitrogruppe, eine Cyanogruppe, eine Azidogruppe, eine Trihalogenomethylgruppe, eine geradkettige oder verzweigte (C₁-C₆)-Al-kylgruppe, eine geradkettige oder verzweigte (C₁-C₆)-Alkoxygruppe, eine Aminogruppe (die gegebenenfalls durch eine geradkettige oder verzweigte (C₁-C₆)-Acylgruppe substituiert ist),
- oder, wenn sie anzwei benachbarten Kohlenstoffatomen vorliegen, gemeinsam mit den Kohlenstoffatomen, an die sie gebunden sind, einen Phenylring oder einen heterocyclischen Ring mit 5 oder 6 Kettengliedern und 1 bis 3 Heteroatomen,
R eine geradkettige oder verzweigte (C₁-C₆)-Alkylgruppe, eine Trihalogenomethylgruppe, eine Phenylgruppe (die gegebenenfalls durch ein oder mehrere Halogenatome oder geradkettige oder verzweigte (C₁-C₆)-Alkylgruppen, Trihalogenomethylgruppen oder geradkettige oder verzweigte (C₁-C₆)-Alkoxygruppen substituiert ist), eine 2-Thienylgruppe (die gegebenenfalls durch ein Halogenatom substituiert ist), eine Naphthylgruppe oder eine Styrylgruppe, und
A eine CH-Gruppe oder ein Stickstoffatom bedeuten, deren Enantiomere, Diastereoisomere und Epimere sowie deren Additionssalze mit einer pharmazeutisch annehmbaren Base.

2. Verbindungen der Formel (I) nach Anspruch 1, worin R eine Trihalogenomethylgruppe bedeutet.

3. Verbindungen der Formel (I) nach Anspruch 1, worin mindestens eine der Gruppen X, Y oder Z eine Nitrogruppe darstellt.

4. Verbindungen der Formel (I) nach Anspruch 1, worin mindestens eine der Gruppen X, Y oder Z ein Chloratom bedeutet.

5. Verbindungen der Formel (I) nach Anspruch 1, worin X, Y oder Z, wenn sie an zwei benachbarten Kohlenstoffatomen vorliegen. gemeinsam mit den Kohlenstoffatomen, an die sie gebunden sind. einen 1,2,5-Oxadiazolring bilden.

6. Verbindung der Formel (I) nach Anspruch 1, nämlich 7-Nitro-3-(trifluorme-thylsulfonylamino)-2-(1H)-chinolinon.

7. Verfahren zur Herstellung der Verbindungen der Formel (I), **dadurch gekennzeichnet**, daß man als Ausgangsprodukt eine Verbindung der Formel (II): 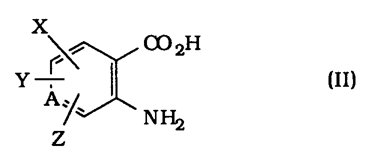 in der X, Y, Z und A die bezüglich der Formel (I) angegebenen Bedeutungen besitzen, verwendet, welches man in Abhängigkeit von der Art der Substituenten X, Y und Z
entweder in aprotischem Medium in Gegenwart von Lithiumaluminiumhydrid, Aluminiumhydrid, Diboran oder Borankomplexen oder in saurem protischem Medium, wenn man Natriumcyanoborhydrid verwendet,
reduziert zur Bildung eines Alkohols der Formel (III): 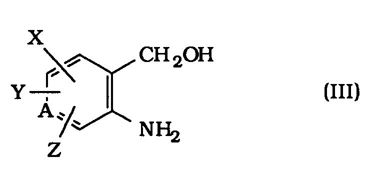 in der X, Y, Z und A die bezüglich der Formel (I) angegebenen Bedeutungen besitzen,
welchen man mit Hilfe eines Metalloxids in einem inerten Lösungsmittel, eines Alkalimetallhalogenats in einem protischen Lösungsmittel oder eines Säurechlorids in Dimethylsulfoxid,
oxidiert zur Bildung des Aldehyds der Formel (IV): 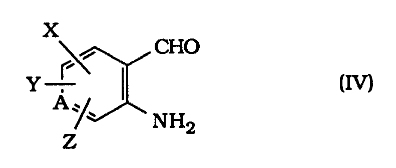 in der X, Y, Z und A die bezüglich der Formel (I) angegebenen Bedeutungen besitzen,
der anschließend
1°/entweder:
mit einem Malonsäurealkylester der Formel (V) 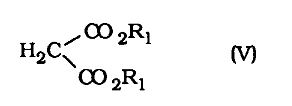 in der R₁ eine geradkettige oder verzweigte (C₁-C₆)-Alkylgruppe, die gegebenenfalls durch eine oder mehrere Phenylgruppen substituiert ist, bedeutet, in protischem Medium in Gegenwart eines Alkalimetallalkoholats, eines tertiären Amins oder eines Alkalimetallhydroxids kondensiert
zur Bildung eines 2-(1H)-Chinolinons der Formel (VI): 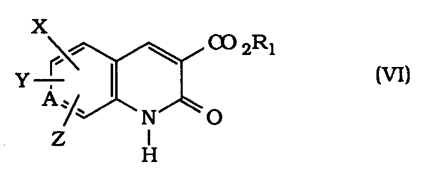 in der X, Y, Z, A und R₁ die oben angegebenen Bedeutungen besitzen, welches:
- durch Reaktion mit einem Trialkyloxoniumtetrafluorborat in Ether oder-Dichlormethan,
- oder durch Umsetzen mit einem Phosphoroxidhalogenid, einem Phosphorpentahalogenid, einem Thionylhalogenid, Phosgen, Thiophosgen, Imminiumphosgen oder Phosgen-trimerem gefolgt von einer Reaktion des in dieser Weise gebildeten 2-Halogenochinolins mit einem Alkalimetallalkoxid der Formel R'₁OM (worin R'₁ die gleichen Bedeutungen besitzt wie R₁ und M ein Alkalimetall darstellt) in alkoholischem Medium,
- oder durch Reaktion mit Natriumhydrid, Butyllithium, einem Alkalimetallalkoholat in Gegenwart eines Alkylierungsmittels der Formel R'₁V (worin R'₁ die oben angegebenen Bedeutungen besitzt und V eine austretende Gruppe darstellt),
geschützt wird, so daß man ein 2-Alkoxychinolin der Formel (VII): 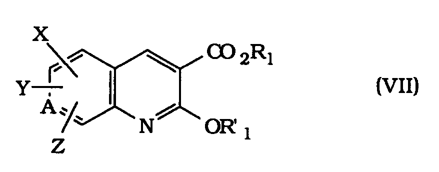 in der X, Y, Z, A, R₁ und R'₁ die oben angegebenen Bedeutungen besitzen, erhält,
welches in Gegenwart eines Alkalimetallhydroxids in wäßrigem oder wäßrig-alkoholischem Medium hydrolysiert wird,
zur Bildung der Säure der Formel (VIII): 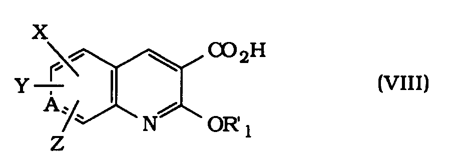 in der X, Y, Z, A und R'₁ die oben angegebenen Bedeutungen besitzen, welche:
a) in das entsprechende Säureazid umgewandelt wird:
- durch Aktivieren der Säurefunktion in Form des Säureanhydrids oder des Säurehalogenids und anschließende Reaktion mit Natriumazid in einem inerten Lösungsmittel,
- oder durch Umsetzen mit Diphenoxyphosphorylazid in Gegenwart eines tertiären Amins in einem inerten Lösungsmittel,
b) umgelagert wird durch Erhitzen in einem Inerten Lösungsmittel in wasserfreiem Medium unter Zugabe eines Überschusses eines Alkohols der Formel R''₁OH (worin R''₁ die gleichen Bedeutungen besitzt wie R₁) bis zur Beendigung der Gasentwicklung, oder durch direktes Erhitzen in dem Alkohol R''₁OH,
so daß man das entsprechende Carbamat der Formel (IX): 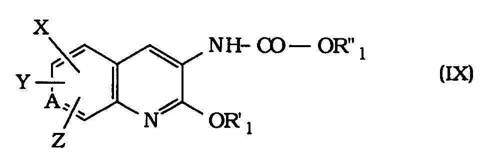 in der X, Y, Z, A, R'₁, R''₁ die oben angegebenen Bedeutungen besitzen, erhält, welches entweder in wasserfreiem saurem Medium hydrolysiert wird oder in wasserfreiem Medium mit Wasserstoff in Gegenwart eines Katalysators hydriert wird,
so daß man das Amin der Formel (X): 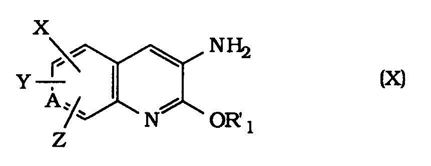 in der X, Y, Z, A und R'₁ die oben angegebenen Bedeutungen besitzen, erhält,
welches einer Sulfonylierung unterworfen wird durch Umsetzen mit einem Sulfonylhalogenid oder einem Sulfonylanhydrid der Formel RSO₂W bzw. (RSO₂)₂O (worin R die gleichen Bedeutungen besitzt wie die bezüglich der Formel (I) angegebenen und W ein Halogenatom darstellt) in Gegenwart eines tertiären Amins und in einem aprotischen Lösungsmittel,
so daß man das Sulfonamid der Formel (XI): 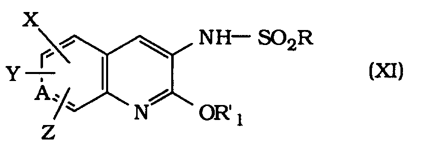 in der X, Y, Z, A, R und R'₁ die oben angegebenen Bedeutungen besitzen, erhält,
von welchem die Schutzgruppe in Abhängigkeit von der Art der Gruppe R'₁ durch Erhitzen in wäßrigem saurem Medium oder durch katalytische Hydrierung abgespalten wird, so daß man die Verbindung der Formel (I) erhält,
2°/oder:
mit einem Alkylnitroacetat in protischem Medium in Gegenwart eines Alkalimetallalkoholats, eines tertiären Amins oder eines Alkalimetallhydroxids, oder in inertem Medium mit hohem Siedepunkt in Gegenwart eines tertiären oder sekundären Amins bei der Rückflußtemperatur des Lösungsmittels kondensiert wird,
so daß man das 3-Nitro-2-(1H)-chinolinon der Formel (XII): 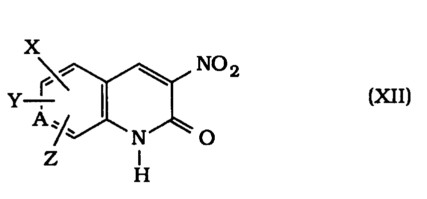 in der X, Y, Z und A die bezüglich der Formel (I) angegebenen Bedeutungen besitzen, erhält,
welches mit Hilfe der bezüglich des Schutzes des Chinolinons der Formel (VI) zu dem 2-Alkoxychinolin der Formel (VII) angegebenen Methoden geschützt wird,
so daß man das 2-Alkoxychinolin der Formel (XIII): 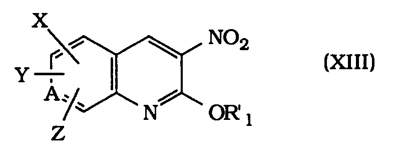 in der X, Y, Z, A und R'₁ die bezüglich der Formel (I) angegebenen Bedeutungen besitzen, erhält,
dessen Nitrogruppe in Gegenwart von Zinnchlorid in einem Alkohol, von Natriumhydrosulfit in Wasser oder durch katalytische Hydrierung reduziert wird,
so daß man das oben definierte Amin der Formel (X) erhält,
welches man einer Sulfonierung und der Abspaltung der Schutzgruppen, wie sie oben beschrieben worden sind, unterwirft, zur Bildung einer Verbindung der Formel (I),
3°/oder:
mit einem geschützten Glycinderivat der Formel (XIV), das in Form des entsprechenden Anhydrids, Azids, Cyanids oder Halogenids aktiviert ist, in inertem Medium und in Gegenwart von Triethylamin der Formel: 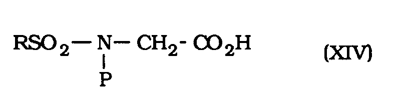 in der R die bezüglich der Formel (I) angegebenen Bedeutungen besitzt und P eine klassische Schutzgruppe der Aminogruppe einer Aminosäure darstellt, kondensiert wird,
zur Bildung des Aldehyds der Formel (XV): 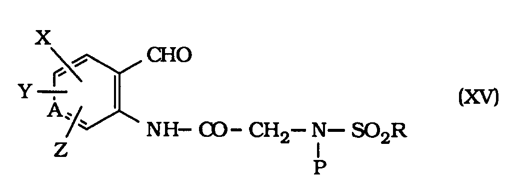 in der X, Y, Z, A, P und R die oben angegebenen Bedeutungen besitzen, welcher in alkoholischem Medium in Gegenwart eines Alkalimetallalkoholats, eines tertiären Amins oder eines Alkalimetallhydroxids cyclisiert wird.
zur Bildung des Chinolinons der Formel (XVI): 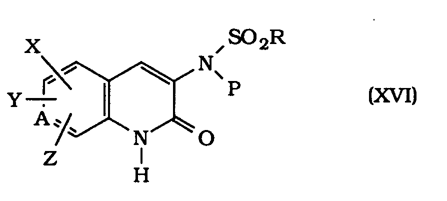 in der X, Y, Z, A, R und P die oben angegebenen Bedeutungen besitzen, von dem in Gegenwart von Aluminiumchlorid in einem inerten Lösungsmittel oder durch katalytische Hydrierung die Schutzgruppe abgespalten wird.
so daß man eine Verbindung der Formel (I) erhält,
welche Verbindung der Formel (I)
- wenn X und/oder Y und/oder Z ein Wasserstoffatom bedeuten, elektrophilen Substitutionen unter Anwendung klassischer Substitutionsmethoden für aromatische Kerne unterworfen wird zur Bildung einer am Phenylrest des Chinolinons mono-, di- oder trisubstituierten Verbindung der Formel (I),
- wenn X, Y oder Z eine Nitrogruppe darstellen, in das entsprechende Aminoderivat oder Acylaminoderivat umgewandelt werden kann, welches Aminoderivat seinerseits mit Hilfe klassischer Methoden der organischen Chemie in das entsprechende Azidoderivat umgewandelt werden kann,
- wenn X eine Nitrogruppe und Y, das am benachbarten Kohlenstoffatom gebunden ist, eine Azidogruppe darstellt, einer Cyclisierung unterworfen werden kann zur Bildung des entsprechenden 1,2,5-Oxadiazolo-Derivats,
- gegebenenfalls mit Hilfe einer klassischen Reinigungsmethode gereinigt werden kann,
- gegebenenfalls mit Hilfe einer klassischen Trennmethode in die Isomeren aufgetrennt werden kann, oder
- gewünschtenfalls in seine Additionssalze mit einer pharmazeutisch annehmbaren Base umgewandelt werden kann.

8. Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien.

9. Pharmazeutische Zubereitungen nach Anspruch 8, enthaltend mindestens einen Wirkstoff nach einem der Ansprüche 1 bis 6, die als Inhibitoren für pathologische Phänomene nützlich sind, die mit einer Hyperaktivierung der Neurotransmissionswege für anregende Aminosäuren geeignet sind.

## Patentansprüche (Patentansprüche für folgende(n) Vertragsstaat(en): ES)

1. Verfahren zur Herstellung von Verbindungen der Formel (I): 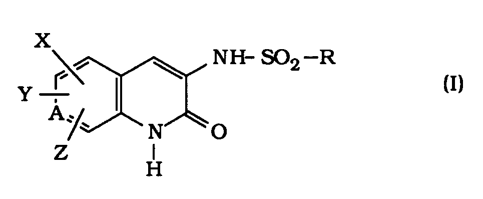 in der:
X, Y, Z:
- die gleichartig oder verschieden sein können, ein Wasserstoffatom, ein Halogenatom, eine Nitrogruppe, eine Cyanogruppe, eine Azidogruppe, eine Trihalogenomethylgruppe, eine geradkettige oder verzweigte (C₁-C₆)-Al-kylgruppe, eine geradkettige oder verzweigte (C₁-C₆)-Alkoxygruppe, eine Aminogruppe (die gegebenenfalls durch eine geradkettige oder verzweigte (C₁-C₆)-Acylgruppe substituiert ist),
- oder, wenn sie an zwei benachbarten Kohlenstoffatomen vorliegen, gemeinsam mit den Kohlenstoffatomen, an die sie gebunden sind, einen Phenylring oder einen heterocyclischen Ring mit 5 oder 6 Kettengliedern und 1 bis 3 Heteroatomen,
R eine geradkettige oder verzweigte (C₁-C₆)-Alkylgruppe, eine Trihalogenomethylgruppe, eine Phenylgruppe (die gegebenenfalls durch ein oder mehrere Halogenatome oder geradkettige oder verzweigte (C₁-C₆)-Alkylgruppen, Trihalogenomethylgruppen oder geradkettige oder verzweigte (C₁-C₆)-Alkoxygruppen substituiert Ist), eine 2-Thienylgruppe (die gegebenenfalls durch ein Halogenatom substituiert ist), eine Naphthylgruppe oder eine Styrylgruppe, und
A eine CH-Gruppe oder ein Stickstoffatom bedeuten,
und von deren Enantiomeren, Diastereoisomeren und Epimeren sowie von deren Additionssalzen mit einer pharmazeutisch annehmbaren Base**, dadurch gekennzeichnet**, daß man als Ausgangsprodukt eine Verbindung der Formel (II): 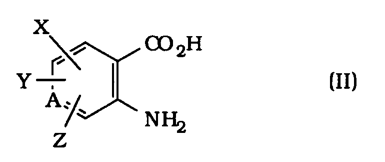 in der X, Y, Z und A die bezüglich der Formel (I) angegebenen Bedeutungen besitzen, verwendet, welches man in Abhängigkeit von der Art der Substituenten X, Y und Z
entweder in aprotischem Medium in Gegenwart von Lithiumaluminiumhydrid, Aluminiumhydrid, Diboran oder Borankomplexen oder in saurem protischem Medium, wenn man Natriumcyanoborhydrid verwendet,
reduziert zur Bildung eines Alkohols der Formel (III): 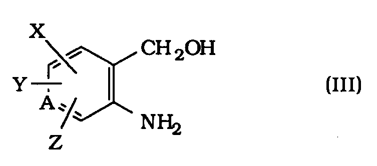 in der X, Y, Z und A die bezüglich der Formel (I) angegebenen Bedeutungen besitzen,
welchen man mit Hilfe eines Metalloxids in einem inerten Lösungsmittel, eines Alkalimetallhalogenats in einem protischen Lösungsmittel oder eines Säurechlorids in Dimethylsulfoxid,
oxidiert zur Bildung des Aldehyds der Formel (IV): 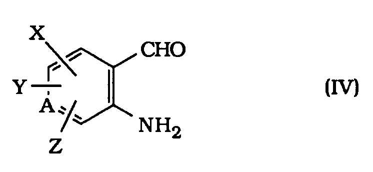 in der X, Y, Z und A die bezüglich der Formel (I) angegebenen Bedeutungen besitzen,
der anschließend
1°/entweder:
mit einem Malonsäurealkylester der Formel (V) 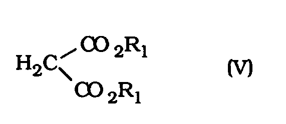 in der R₁ eine geradkettige oder verzweigte (C₁-C₆)-Alkylgruppe, die gegebenenfalls durch eine oder mehrere Phenylgruppen substituiert ist, bedeutet, in protischem Medium in Gegenwart eines Alkalimetallalkoholats, eines tertiären Amins oder eines Alkalimetallhydroxids kondensiert
zur Bildung eines 2-(1H)-Chinolinons der Formel (VI): 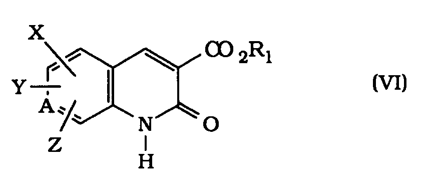 in der X, Y, Z, A und R₁ die oben angegebenen Bedeutungen besitzen, welches:
- durch Reaktion mit einem Trialkyloxoniumtetrafluorborat in Ether oder-Dichlormethan,
- oder durch Umsetzen mit einem Phosphoroxidhalogenid, einem Phosphorpentahalogenid, einem Thionylhalogenid` Phosgen, Thiophosgen, Imminiumphosgen oder Phosgen-trimerem gefolgt von einer Reaktion des in dieser Weise gebildeten 2-Halogenochinolins mit einem Alkalimetallalkoxid der Formel R'₁OM (worin R'₁ die gleichen Bedeutungen besitzt wie R₁ und M ein Alkalimetall darstellt) in alkoholischem Medium,
- oder durch Reaktion mit Natriumhydrid, Butyllithium, einem Alkalimetallalkoholat in Gegenwart eines Alkylierungsmittels der Formel R'₁V (worin R'₁ die oben angegebenen Bedeutungen besitzt und V eine austretende Gruppe darstellt),
geschützt wird, so daß man ein 2-Alkoxychinolin der Formel (VII): 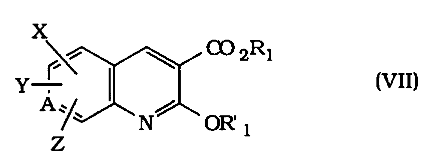 in der X, Y, Z, A, R₁ und R'₁ die oben angegebenen Bedeutungen besitzen, erhält,
welches in Gegenwart eines Alkalimetallhydroxids in wäßrigem oder wäßrig-alkoholischem Medium hydrolysiert wird,
zur Bildung der Säure der Formel (VIII): 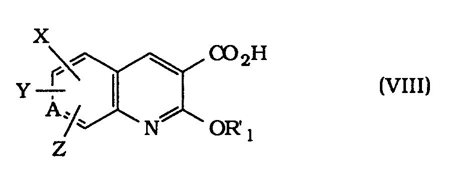 in der X, Y, Z, A und R'₁ die oben angegebenen Bedeutungen besitzen, welche:
a) in das entsprechende Säureazid umgewandelt wird:
- durch Aktivieren der Säurefunktion in Form des Säureanhydrids oder des Säurehalogenids und anschließende Reaktion mit Natriumazid in einem Inerten Lösungsmittel,
- oder durch Umsetzen mit Diphenoxyphosphorylazid in Gegenwart eines tertiären Amins in einem inerten Lösungsmittel,
b) umgelagert wird durch Erhitzen in einem inerten Lösungsmittel in wasserfreiem Medium unter Zugabe eines Überschusses eines Alkohols der Formel R''₁OH (worin R''₁ die gleichen Bedeutungen besitzt wie R₁) bis zur Beendigung der Gasentwicklung, oder durch direktes Erhitzen in dem Alkohol R''₁OH,
so daß man das entsprechende Carbamat der Formel (IX): 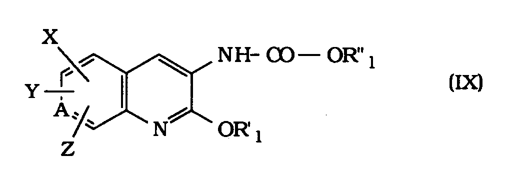 in der X, Y, Z, A, R'₁, R''₁ die oben angegebenen Bedeutungen besitzen, erhält, welches entweder in wasserfreiem saurem Medium hydrolysiert wird oder in wasserfreiem Medium mit Wasserstoff in Gegenwart eines Katalysators hydriert wird,
so daß man das Amin der Formel (X): 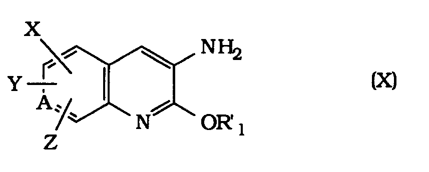 in der X, Y, Z, A und R'₁ die oben angegebenen Bedeutungen besitzen, erhält,
welches einer Sulfonylierung unterworfen wird durch Umsetzen mit einem Sulfonylhalogenid oder einem Sulfonylanhydrid der Formel RSO₂W bzw. (RSO₂)₂O (worin R die gleichen Bedeutungen besitzt wie die bezüglich der Formel (I) angegebenen und W ein Halogenatom darstellt) in Gegenwart eines tertiären Amins und in einem aprotischen Lösungsmittel,
so daß man das Sulfonamid der Formel (XI): 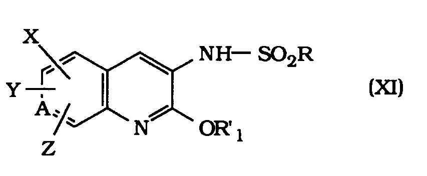 in der X, Y, Z, A, R und R'₁ die oben angegebenen Bedeutungen besitzen, erhält,
von welchem die Schutzgruppe in Abhängigkeit von der Art der Gruppe R'₁ durch Erhitzen in wäßrigem saurem Medium oder durch katalytische Hydrierung abgespalten wird, so daß man die Verbindung der Formel (I) erhält,
2°/oder:
mit einem Alkylnitroacetat in protischem Medium in Gegenwart eines Alkalimetallalkoholats, eines tertiären Amins oder eines Alkalimetallhydroxids, oder in inertem Medium mit hohem Siedepunkt in Gegenwart eines tertiären oder sekundären Amins bei der Rückflußtemperatur des Lösungsmittels kondensiert wird,
so daß man das 3-Nitro-2-(1H)-chinolinon der Formel (XII): 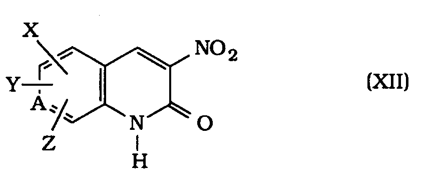 in der X, Y, Z und A die bezüglich der Formel (I) angegebenen Bedeutungen besitzen, erhält,
welches mit Hilfe der bezüglich des Schutzes des Chinolinons der Formel (VI) zu dem 2-Alkoxychinolin der Formel (VII) angegebenen Methoden geschützt wird,
so daß man das 2-Alkoxychinolin der Formel (XIII): 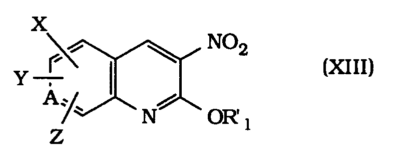 in der X, Y, Z, A und R'₁ die bezüglich der Formel (I) angegebenen Bedeutungen besitzen, erhält,
dessen Nitrogruppe in Gegenwart von Zinnchlorid in einem Alkohol, von Natriumhydrosulfit in Wasser oder durch katalytische Hydrierung reduziert wird,
so daß man das oben definierte Amin der Formel (X) erhält,
welches man einer Sulfonierung und der Abspaltung der Schutzgruppen, wie sie oben beschrieben worden sind, unterwirft, zur Bildung einer Verbindung der Formel (I),
3°/oder:
mit einem geschützten Glycinderivat der Formel (XIV), das in Form des entsprechenden Anhydrids, Azids, Cyanids oder Halogenids aktiviert ist, in inertem Medium und in Gegenwart von Triethylamin der Formel: 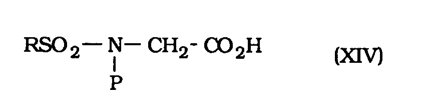 in der R die bezüglich der Formel (I) angegebenen Bedeutungen besitzt und P eine klassische Schutzgruppe der Aminogruppe einer Aminosäure darstellt, kondensiert wird,
zur Bildung des Aldehyds der Formel (XV): 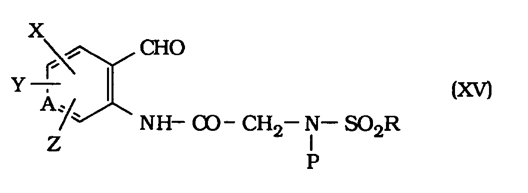 in der X, Y, Z, A, P und R die oben angegebenen Bedeutungen besitzen, welcher in alkoholischem Medium in Gegenwart eines Alkalimetallalkoholats, eines tertiären Amins oder eines Alkalimetallhydroxids cyclisiert wird,
zur Bildung des Chinolinons der Formel (XVI): 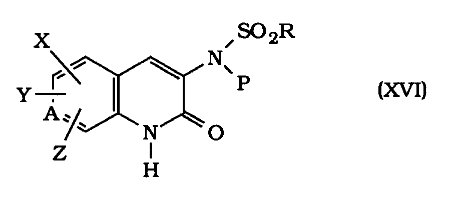 in der X, Y, Z, A, R und P die oben angegebenen Bedeutungen besitzen, von dem in Gegenwart von Aluminiumchlorid in einem inerten Lösungsmittel oder durch katalytische Hydrierung die Schutzgruppe abgespalten wird,
so daß man eine Verbindung der Formel (I) erhält,
welche Verbindung der Formel (I)
- wenn X und/oder Y und/oder Z ein Wasserstoffatom bedeuten, elektrophilen Substitutionen unter Anwendung klassischer Substitutionsmethoden für aromatische Kerne unterworfen wird zur Bildung einer am Phenylrest des Chinolinons mono-, di- oder trisubstituierten Verbindung der Formel (I),
- wenn X, Y oder Z eine Nitrogruppe darstellen, in das entsprechende Aminoderivat oder Acylaminoderivat umgewandelt werden kann, welches Aminoderivat seinerseits mit Hilfe klassischer Methoden der organischen Chemie in das entsprechende Azidoderivat umgewandelt werden kann,
- wenn X eine Nitrogruppe und Y, das am benachbarten Kohlenstoffatom gebunden ist, eine Azidogruppe darstellt, einer Cyclisierung unterworfen werden kann zur Bildung des entsprechenden 1,2,5-Oxadiazolo-Derivats,
- gegebenenfalls mit Hilfe einer klassischen Reinigungsmethode gereinigt werden kann,
- gegebenenfalls mit Hilfe einer klassischen Trennmethode in die Isomeren aufgetrennt werden kann, oder
- gewünschtenfalls in seine Additionssalze mit einer pharmazeutisch annehmbaren Base umgewandelt werden kann.

2. Verfahren zur Herstellung von Verbindungen der Formel (I) nach Anspruch 1, worin R eine Trihalogenomethylgruppe darstellt.

3. Verfahren zur Herstellung von Verbindungen der Formel (I) nach Anspruch 1, worin mindestens eine der Gruppen X, Y oder Z eine Nitrogruppe bedeutet.

4. Verfahren zur Herstellung der Verbindungen der Formel (I) nach Anspruch 1, worin mindestens eine der Gruppen X, Y oder Z ein Chloratom bedeutet.

5. Verfahren zur Herstellung der Verbindungen der Formel (I) nach Anspruch 1, worin X, Y oder Z, wenn sie an zwei benachbarten Kohlenstoffatomen gebunden sind, gemeinsam mit den Kohlenstoffatomen, an die sie gebunden sind, einen 1,2,5-Oxadiazolring bilden.

6. Verfahren zur Herstellung der Verbindung der Formel (I) nach Anspruch 1, nämlich 7-Nitro-3-(trifluormethylsulfonylamino)-2-(1H)-chinolinon.
